(19) Europäisches Patentamt
European Patent Office
Office européen des brevets

(11) Veröffentlichungsnummer: **0 321 864**
**A2**

# (12) EUROPÄISCHE PATENTANMELDUNG

(21) Anmeldenummer: **88121011.6**

(22) Anmeldetag: **15.12.88**

(51) Int. Cl.⁴: **C07D 413/12 , C07D 265/36 , C07C 127/19 , C07C 95/08 , C07D 213/38 , C07D 233/61 , C07C 103/183 , C07D 213/20 , A61K 31/535 , A61K 31/14**

(30) Priorität: **19.12.87 DE 3743265**

(43) Veröffentlichungstag der Anmeldung:
**28.06.89 Patentblatt 89/26**

(84) Benannte Vertragsstaaten:
**AT BE CH DE ES FR GB GR IT LI LU NL SE**

(71) Anmelder: **BOEHRINGER INGELHEIM KG**
**Postfach 200**
**D-6507 Ingelheim am Rhein(DE)**

(84) **BE CH DE ES FR GR IT LI LU NL SE AT**

Anmelder: **BOEHRINGER INGELHEIM INTERNATIONAL G.M.B.H.**
**Postfach 20**
**D-6507 Ingelheim am Rhein(DE)**

(84) **GB**

(72) Erfinder: **Schromm, Kurt, Dr.**
**In der Dörrwiese 35**
**D-6507 Ingelheim(DE)**
Erfinder: **Mentrup, Anton, Dr.**
**Biebricher Allee 15**
**D-6200 Wiesbaden(DE)**
Erfinder: **Renth, Ernst-Otto, Dr.**
**Frankenstrasse 11**
**D-6507 Ingelheim am Rhein(DE)**
Erfinder: **Muacevic, Gojko, Dr.**
**In der Dörrwiese 13**
**D-6507 Ingelheim am Rhein(DE)**
Erfinder: **Traunecker, Werner, Dr.**
**Birkenweg 1**
**D-6538 Münster-Sarmsheim(DE)**

## (54) Neue Ammoniumverbindungen, ihre Herstellung und Verwendung.

(57) Die neuen Verbindungen der Formel

$$Q - \underset{OH}{CH} - \overset{R_4}{CH} - NH - \overset{R_5}{\underset{R_6}{C}} - (CH_2)_n - R \qquad (I).$$

(die Bedeutung der Symbole ist in der Beschreibung angegeben) sind nach üblichen Verfahren in an sich bekannter Weise herstellbar; sie können als Arzneimittelwirkstoffe verwendet werden.

EP 0 321 864 A2

## Neue Ammoniumverbindungen, ihre Herstellung und Verwendung

Die Erfindung betrifft neue quartäre Ammoniumverbindungen, die nach an sich bekannten Verfahren hergestellt und als Arzneistoffe, insbesondere für die inhalative Anwendung, verwendet werden können.

Die neuen Verbindungen entsprechen der Formel

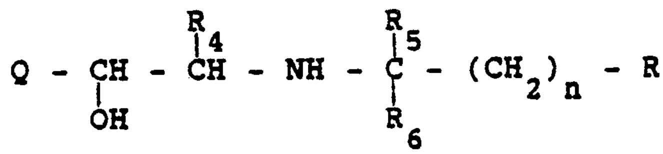

(I).

In dieser Formel bedeutet:

Q: den substituierten Phenylrest broncholytisch wirksamer Verbindungen;

R: einen Rest, z.B. einen Alkoxy-, Arylalkoxy-, Aryloxyalkoxy-, Aryl-, Aryloxy-, Arylcarbonamidorest, einen heterocyclischen Rest oder heterocyclisch substituierten Carbonamidorest, der eine quartäre Ammoniumgruppierung enthält,

$R_4$: H, $CH_3$, $C_2H_5$;

$R_5$: H, $CH_3$;

$R_6$: H, $CH_3$;

n: 1, 2, 3, 4 oder 5.

Es wurde gefunden, daß die Einführung einer quartären Ammoniumgruppe an geeigneter Stelle in das Molekül bekannter inhalativ wirksamer Broncholytika es ermöglichst, bei weitgehender Erhaltung der broncholytischen (topischen) Wirkung die störenden systemischen Nebenwirkungen größtenteils auszuschalten. Wie sich gezeigt hat, kann die Art der quartären Ammoniumgruppierung innerhalb eines großen Variationsbereichs gewählt werden, ohne daß die erfindungsgemäße Differenzierung von gewünschten und unerwünschten Wirkungen entscheidend beeinträchtigt würde.

Die neuen Verbindungen lassen sich im wesentlichen durch die Formel

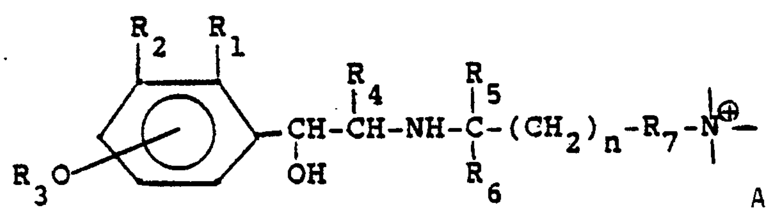

(Ia)

wiedergegeben.

Soweit nichts anderes angegeben ist, bedeutet in dieser Formel und im folgenden n: 1, 2, 3, 4 oder 5;

$R_1$: H, $CH_3$, $OCH_3$, Cl, F;

$R_2$: H, $R_3O$, $CH_2OH$, NHCHO, $NHCOCH_3$, $NHSO_2CH_3$, $NHCONH_2$;

$R_3$: H, Acyl, N,N-Dialkylcarbamoyl, wobei sich die Gruppe $R_3O$ in 4- oder 5-Stellung befindet;

die Gruppe

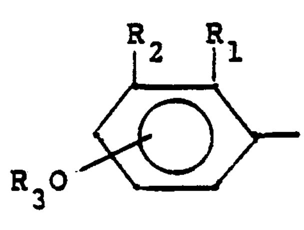

(II)

steht außerdem für

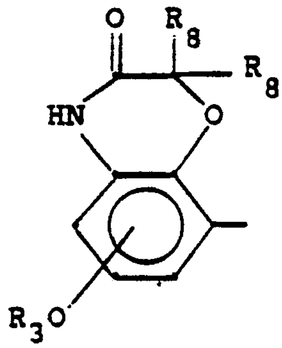 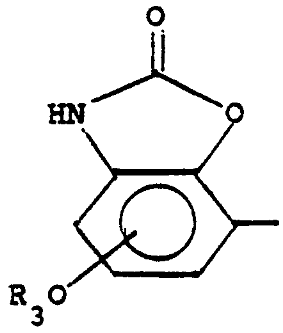 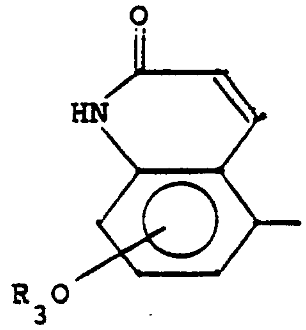

(IIa) (IIb) (IIc)

$R_4$: H, $CH_3$, $C_2H_5$;
$R_5$: H, $CH_3$;
$R_6$: H, $CH_3$;
$R_7$: eine Einfachbindung oder ein zweibindiges Brückenglied, das auch über Ringatome eines Heterocyclus an den Ammoniumstickstoff gebunden sein kann;
$R_8$: H oder $CH_3$;
$-N^{\oplus}-$: eine quartäre Ammoniumgruppe.
$An^{\ominus}$: 1 Äquivalent eines Anions

Die Gruppierung $-R_7-N^{\oplus}-$ steht (während n und $R_1$ bis $R_6$ die obige Bedeutung haben), hauptsächlich für

-E (III a)
-Ar-B-E (III) b)
-O-Ar-B-E (III c)
-NH-CO-E (III d)
-NH-CO-Ar-B-E (III e)
$-O-(C_mH_{2m})$-A-E (III f)
$-O-(C_mH_{2m})$-A-Ar-B-E (III g)

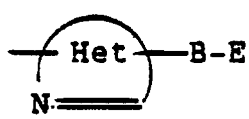

(III h)

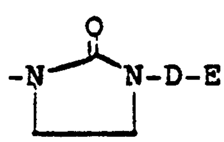

(III i)

Dabei bedeuten

m: 2, 3, 4, 5 oder 6;
A: eine Einfachbindung oder NH-CO-($C_1$-$C_4$)-Alkylen;
B: eine Einfachbindung oder -O-($C_1$-$C_4$)-Alkylen-, -NH-CO-($C_1$-$C_4$)-Alkylen-, -($C_1$-$C_4$)-Alkylen-;
D: -($C_1$-$C_4$)-Alkylen-
E: die Gruppen

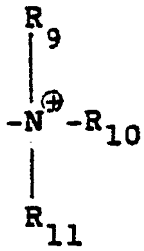 oder 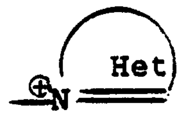 oder 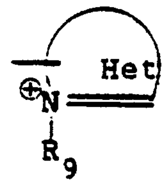

Ar: Arylen, insbesondere unsubstituiertes oder substituiertes Phenylen oder Naphthylen;

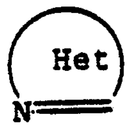 :

N-Heterocyclen, die mit einem Benzolring kondensiert und unsubstituiert oder substituiert sein können und die gegebenenfalls ein oder mehrere zusätzliche Heteroatome im Ring enthalten können;

$R_9$: $(C_1-C_4)$-Alkyl;

$R_{10}$: $(C_1-C_4)$-Alkyl;

$R_{11}$: $(C_1-C_4)$-Alkyl, $(C_1-C_4)$-Alkylen-$COO^{\ominus}$, $(C_1-C_4)$-Alkylen-$SO_3^{\ominus}$, $(C_1-C_4)$-Alkylen -OH, $(C_3-C_6)$-Cycloalkyl, $R_9$ und $R_{10}$ gemeinsam auch $(C_4-C_6)$-Alkylen.

Hervorzuheben sind (für $-R_7-N^{\oplus}$):

$-N^{\oplus}R_9R_{10}R_{11}$ (III a 1)

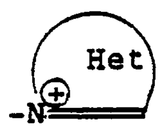 (III a 2)

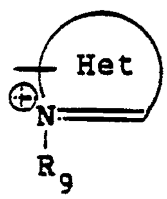 (III a 3)

$-Ar-B-N^{\oplus}R_9R_{10}R_{11}$ (III b 1)

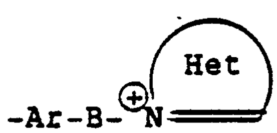 (III b 2)

$-O-Ar-B-N^{\oplus}R_9R_{10}R_{11}$ (III c 1)

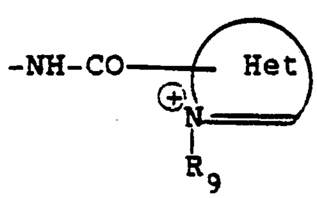 (III d 1)

$-NH-CO-Ar-B-N^{\oplus}R_9R_{10}R_{11}$ (III e 1)

$-O-(C_mH_{2m})-A-N^{\oplus}R_9R_{10}R_{11}$ (III f 1)

$-O-(C_mH_{2m})-A-Ar-B-N^{\oplus}R_9R_{10}R_{11}$ (III g 1)

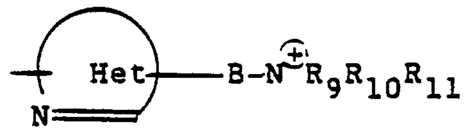

(III h 1)

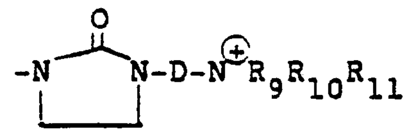

(III i )

Die erfindungsgemäßen Verbindungen können als Enantiomerengemische, insbesondere als Racemate, gegebenenfalls auch Diastereomerenpaare, und als reine Enantiomere vorliegen, jeweils auch als Salze mit (vorzugsweise physiologisch verträglichen) Säuren.

Die Alkyl- und Alkylengruppen in den obigen Definitionen können geradkettig oder verzweigt sein. Soweit keine näheren Angaben gemacht sind, enthalten sie 1 bis 6, vorzugsweise 1 bis 4, insbesondere 1 bis 2 C-Atome. Dies gilt auch für die C-Ketten, die Bestandteil anderer Reste sind. Als Substituenten in Aryl(en) kommen vor allem F, Cl, $CH_3$, $CH_3O$ in Betracht, wobei mit "Aryl" bzw. "Arylen" die entsprechenden von Benzol bzw. Naphthalin abgeleiteten Reste gemeint sind. "Acylreste" sind hier Carbonsäurereste mit bis zu 7 C-Atomen, insbesondere Acetyl. Die Brücke $R_7$ kann mit dem N-Atom der quartären Ammoniumgruppe verknüpft sein. Falls die quartäre Ammoniumgruppe Bestandteil eines Heterocyclus ist, kann die Brücke auch mit einem anderen Ringatom des Heterocyclus verbunden sein. Reste der Formel

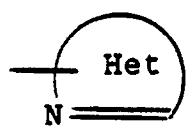

sind vor allem

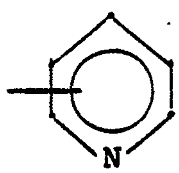 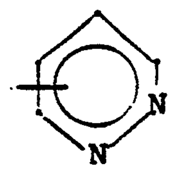 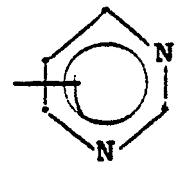 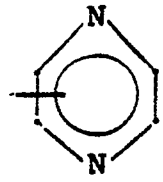

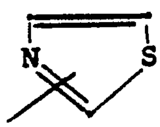 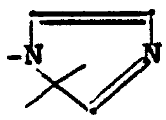 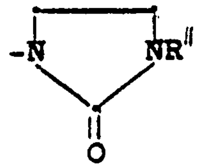

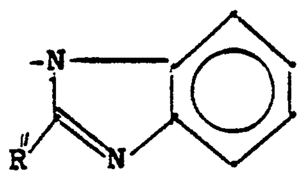 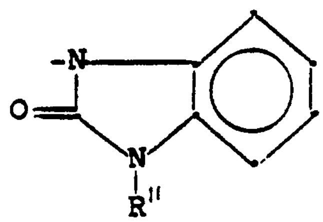

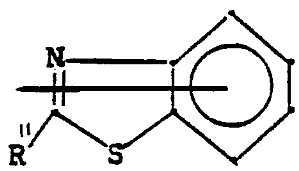 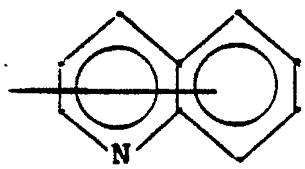 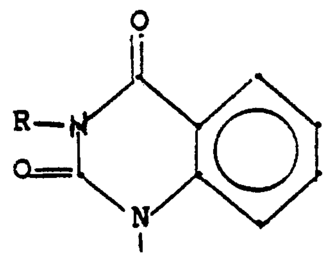

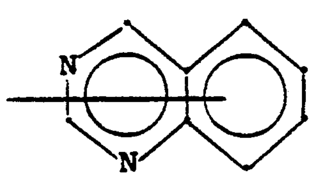 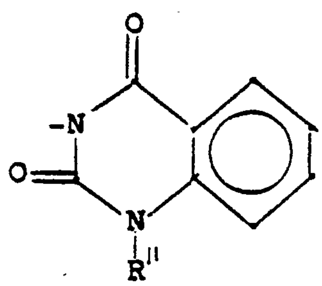 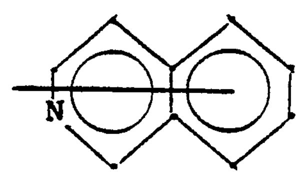

ferner Triazine; R″ gleich H oder $C_1$-$C_4$-Alkyl)

In einer bevorzugten Ausführungsform der Erfindung steht

$R_1$ für H, $CH_3$, $OCH_3$, Cl, F;

$R_2$ für OH oder - im Falle $R_1$ gleich Cl, F -für H;

Typische Beispiele für E sind z.B.

$-N^{\oplus}(CH_3)_3$,

$-N^{\oplus}(CH_3)_2CH_2CH_2CH_2SO_3^{\ominus}$,

$-N^{\oplus}(CH_3)_2-(CH_2)_4-SO_3^{\ominus}$,

$-N^{\oplus}(CH_3)_2CH_2CH_2CO_2^{\ominus}$,

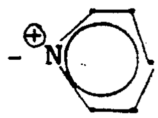 , 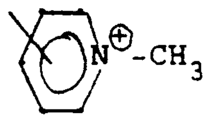 , 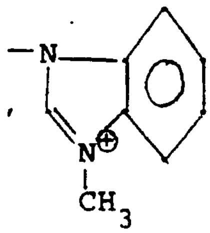

$R_1$ und $R_2$ gemeinsam für

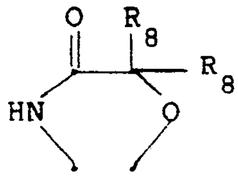 oder 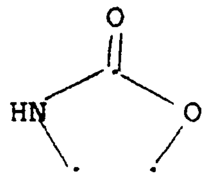 ;

$R_3$ für H, ;
$R_4$ für H, $C_2H_5$;
$R_5$,$R_6$: beide für H oder beide für $CH_3$;
n für 1, 2 oder 3;

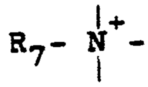 für 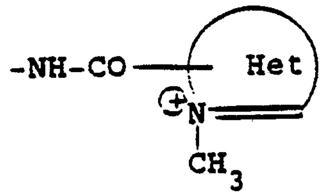

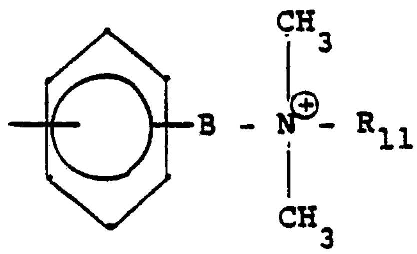 , 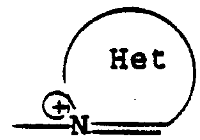 ,

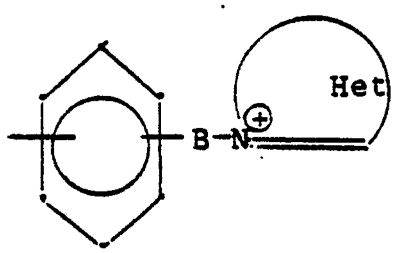

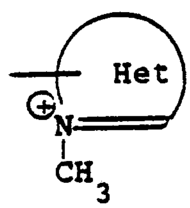 oder 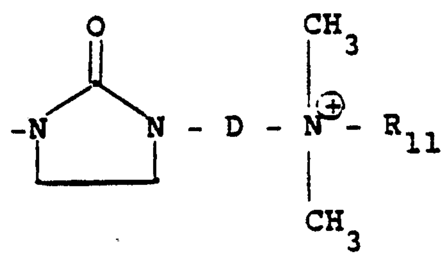

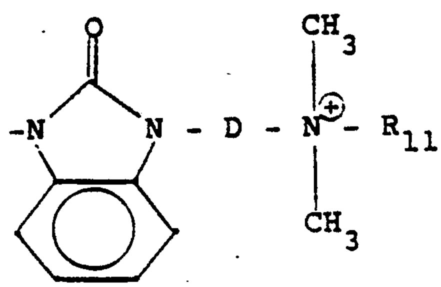 ,

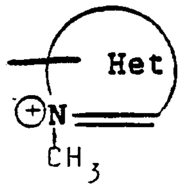 steht hier für 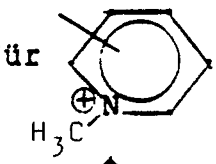 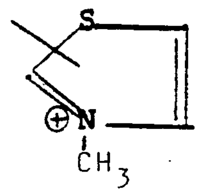

oder 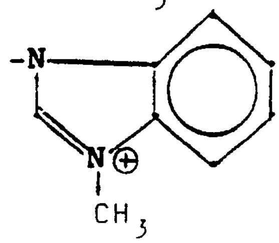

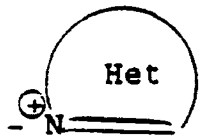 für 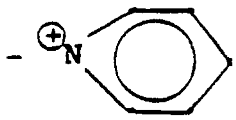

während B, D und $R_{11}$ die obige Bedeutung haben.

Besonders hervorzuheben sind die Verbindungen, in denen sich die nachstehenden Substituentenkombinationen finden:

$R_1/R_2/OR_3$:

(a) $CH_3$ oder $OCH_3/OH/4\text{-}OH$

(b) H/OH/4- oder 5-OH

(c) 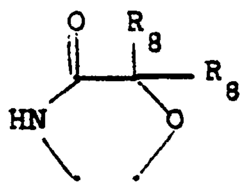 oder 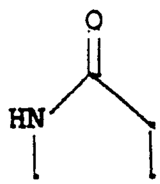

($R_1$ und $R_2$ gemeinsam)/4- oder 5-OH

$R_4$: H, wenn $R_5$ und $R_6$ $CH_3$;

und $C_2H_5$, wenn $R_5$ und $R_6$ H sind;

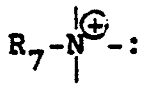

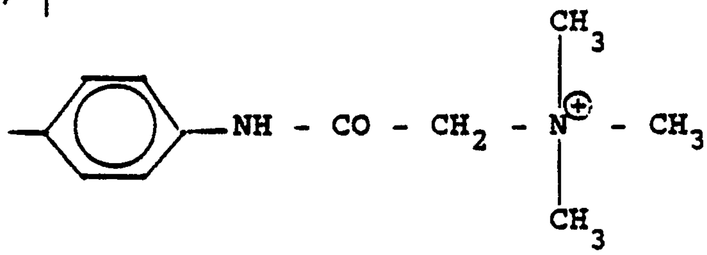

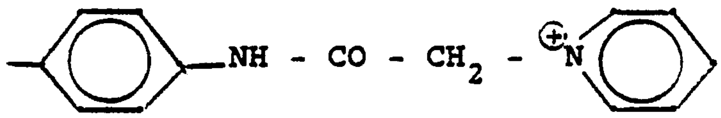

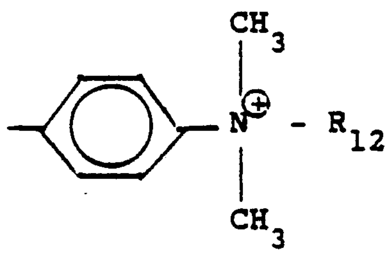

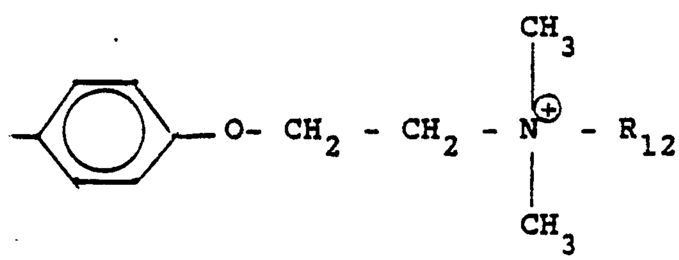

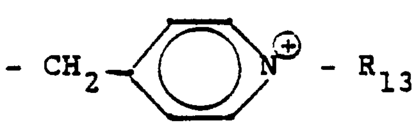

$R_{12}$, $R_{13}$: $CH_3$, $CH_2$-$COO^{\ominus}$, $CH_2$-$CH_2$-$COO^{\ominus}$, -$CH_2$-$CH_2$-$CH_2$-$SO_3^{\ominus}$ ;

Die erfindungsgemäßen Verbindungen können als Enantiomerengemische, insbesondere als Racemate, gegebenenfalls auch Diastereomerenpaare, und als reine Enantiomere vorliegen, jeweils auch als Salze mit (vorzugsweise physiologisch verträglichen) Säuren.

Die neuen Verbindungen können nach an sich bekannten Methoden hergestellt werden:

1. Quaternierung von Verbindungen der Formel

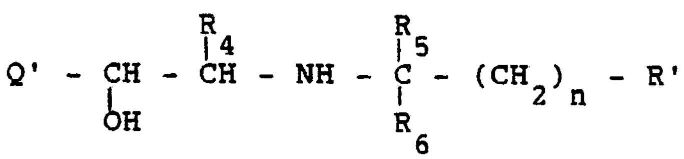 (IV),

in der n, $R_4$, $R_5$ und $R_6$ die obige Bedeutung haben und R′ für einen Rest steht, der mit R übereinstimmt, außer daß er anstelle der quartären Ammoniumgruppe eine tertiäre Aminogruppe enthält und in der Q′ die

gleiche Bedeutung wie Q hat, wobei jedoch etwa darin vorhandene OH-Gruppen, wie auch die zentrale NH-Gruppe, durch hydrogenolytisch abspaltbare Schutzgruppen geschützt sein können, mit geeigneten Alkylierungsmitteln umsetzt und gegebenenfalls vorhandene Schutzgruppen mit Wasserstoff/Katalysator entfernt.

Das Verfahren eignet sich zur Herstellung derjenigen Verbindungen der Formel I, in denen die quartäre Ammoniumgruppe nicht die Form

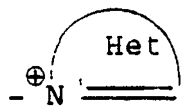

hat.

Zur Einführung von ($C_1$-$C_4$)-Alkylen-$SO_3^{\ominus}$ eignen sich vor allem Cl-($C_1$-$C_4$)-Alkylen-$SO_3Na$ und HO-($C_1$-$C_4$)-Alkylen-$SO_2$-O-($C_1$-$C_4$)-Alkylen-$SO_3Na$.

Die Umsetzung erfolgt zweckmäßig in einem inerten polaren Lösungsmittel bei Raumtemperatur oder erhöhter Temperatur (bis etwa 100 °C).

Die Ausgangsstoffe der Formel IV können ebenfalls nach an sich bekannten Methoden erhalten werden. So können Aminoketone der Formel

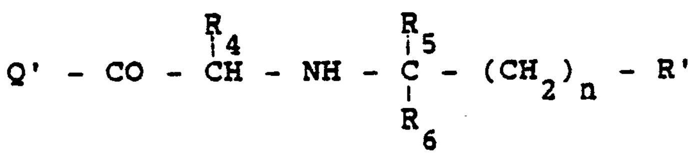

(V),

oder Schiffsche Basen der Formel

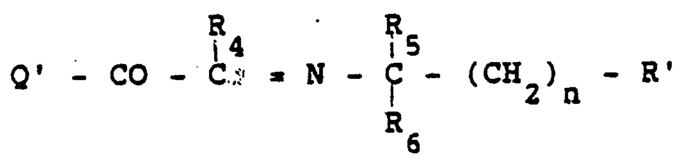

(VI),

worin die Symbole die oben angegebene Bedeutung haben, durch Reduktion mit Wasserstoff und Hydrierungskatalysatoren wie Palladium, Platin oder Raney-Nickel oder mit Hydriden wie NaH in geeigneten Lösungsmitteln, z.B. Ethanol in Verbindungen der Formel IV umgewandelt werden. Schutzgruppen können gewünschtenfalls in üblicher Weise entfernt werden.

2. Zur Herstellung von Verbindungen der Formel

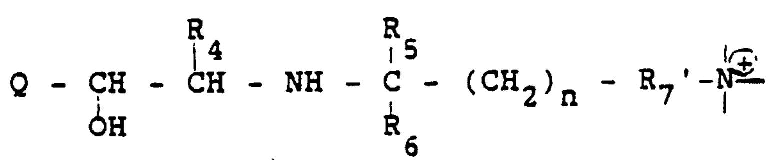

(VII),

worin die Symbole die obige Bedeutung haben, $R_7'$ eine solche Gruppe $R_7$ darstellt, die über ein aliphatisches C-Atom an den quartären Ammoniumstickstoff gebunden ist,

Umsetzung einer Verbindung der Formel

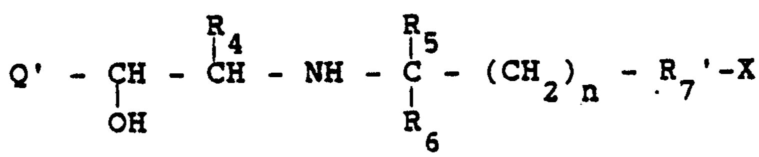

(VIII),

worin die Symbole die obige Bedeutung haben und X eine Gruppe bedeutet, die unter Bindung von $R_7'$ - (und damit Quaternisierung) an das Stickstoffatom eines entsprechenden tertiären Amins abgespalten wird.
Die abspaltbare Gruppe X ist bevorzugt Chlor, Brom oder Jod oder ein Alkyl- oder Arylsulfonsäurerest.
Die Komponenten werden in einem protischen oder aprotischen Lösungsmittel wie Methanol oder Dimethylformamid bei Temperaturen zwischen Raumtemperatur und ca. 100° C zur Reaktion gebracht.
Eventuell vorhandene hydrogenolytisch abspaltbare Schutzgruppen werden nach der Umsetzung nach üblichen Methoden entfernt.
Die Ausgangsstoffe der Formel VIII lassen sich z.B. aus Aminoketonen der Formel

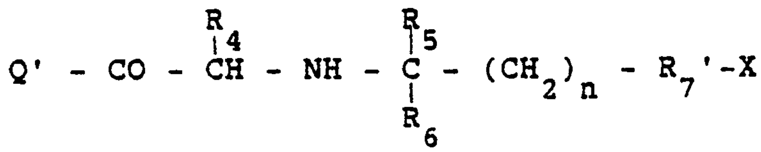
(IX)

mit Hydriden wie Natriumhydrid oder Diboran herstellen.
Die Verbindungen der Formel IX sind ihrerseits nach an sich bekannten Methoden erhältlich.
3. Entfernung von hydrogenolytisch abspaltbaren Schutzgruppen aus einer Verbindung der Formel

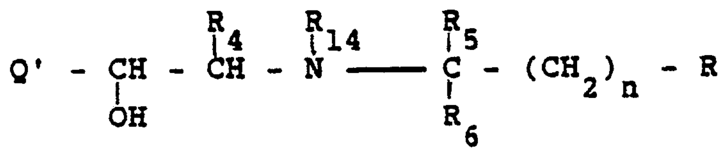
(X)

in der $R_{14}$ für Wasserstoff oder eine hydrogenolytisch abspaltbare Schutzgruppe steht und die übrigen Symbole die oben angegebene Bedeutung haben, wobei die Verbindung X jedoch mindestens eine abzuspaltende Schutzgruppe enthält,
mit Wasserstoff in Gegenwart eines Hydrierungskatalysators.
Die Entfernung der Schutzgruppen wird zweckmäßig mit Palladium als Katalysator in einem inerten Lösungsmittel vorgenommen.
Die Ausgangsstoffe können nach üblichen Methoden, z.B. analog den obigen Verfahren 1 und 2 gewonnen werden.
Die erfindungsgemäßen Verbindungen enthalten mindestens ein asymmetrisches C-Atom. Zur Gewinnung bestimmter Enantiomerer bzw. (im Fall mehrerer Asymmetriezentren) diastereomerer Antipodenpaare verwendet man zweckmäßig Ausgangsstoffe, z.B. der Formel IV oder X, in denen an den betreffenden Asymmetriezentren von vornherein die gewünschte Konfiguration vorliegt.
Die erfindungsgemäßen Verbindungen werden gewünschtenfalls in an sich bekannter Weise in Salze mit physiologisch verträglichen Säuren übergeführt.

Die neuen Verbindungen sind als Arzneistoffe verwendbar. Sie haben vor allem broncholytische, spasmolytische und antiallergische Wirkung, erhöhen die Ciliartätigkeit und verringern entzündlich-exsudative Reaktionen. Sie sind daher u.a. für die Behandlung aller Formen von Asthma und Bronchitis verwendbar.

Die therapeutische und prophylaktische Dosis ist abhängig von der Beschaffenheit und Ernsthaftigkeit des Krankheitszustandes.

Für einen Erwachsenen beträgt die Dosis bei der bevorzugten inhalativen Anwendung 0,001 - 0,5 mg pro die. Die Zubereitung der Präparate erfolgt in der galenisch üblichen Weise mit gebräuchlichen Hilfs- und/oder Trägerstoffen, wobei die erfindungsgemäßen Verbindungen auch mit anderen Wirkstoffen kombiniert werden können, z.B. mit Parasympatholytika (z.B. Ipratropiumbromid, Oxitropiumbromid), Selcretolytika (z.B. Bromhexin, Ambroxol), Antibiotika (z.B. Doxycyclin), Corticosteroiden (z.B. Beclomethason, Flunisolid, Budesonid, Dexamethason, Flumethason, Triamcinolonacetonid und ihre 17- und/oder 21-Ester, etwa Beclomethasondipropionat, ferner die physiologisch verträglichen Salze der genannten Verbindungen), anderen Asthmamitteln wie Dinatriumcromoglicat, Nedocromil und Antiallergika.

Formulierungsbeispiele

1. Inhalationspulver

Mikronisiertes Wirkstoffpulver (Verbindung der Formel I; Teilchengröße ca. 0,5 bis 7µm) werden in einer Menge von 0,02 mg mit 10 mg mikronisierter Lactose und gegebenenfalls geeigneten Mengen weiterer Wirkstoffe in Hartgelatinekapseln abgefüllt. Das Pulver wird aus üblichen Inhalationsgeräten, z.B. gemäß DE-A 3345 722, inhaliert.

2. Dosieraerosol

| | |
|---|---|
| Wirkstoff gemäß Beispiel 8 | 0,1 Gew.-% |
| Sorbitantrioleat | 0,5 Gew.-% |
| Monofluortrichlormethan und Difluordichlormethan (2:3) | 99,4 Gew.-% |

Die Mischung wird in Dosieraerosolgeräte üblicher Art abgefüllt. Die Dosiervorrichtung wird beispielsweise so ausgelegt, daß pro Hub 0,05 ml der Zubereitung abgegeben werden.

Der Vorteil der erfindungsgemäßen Verbindungen besteht darin, daß bei der inhalativen Applikation im Vergleich zu bekannten bronchospasmolytischen β-Mimetika eine besonders ausgeprägte Selektivität im Verhältnis Bronchospasmolyse zu Herzfrequenzsteigerung, positiv inotropen Effekten und Tremor festzustellen ist. Die broncholytische Wirkung wird mit geringen Dosen erzielt, die Wirkung hält lange an.

Im folgenden sind einige pharmakologische Wirkungsangaben für erfindungsgemäße Verbindungen zusammengestellt.

Es wurde die inhalative $EC_{50}$ am wachen, nüchternen Meerschweinchen nach Kallos P. und Pagel, W. (Acta med. scand. 91, 292 (1937)) ermittelt (Histaminspasmus). Die Substanzen wurden in Form einer wäßrigen Lösung geprüft.

| $EC_{50}$ | % |
|---|---|
| A | 0,06 |
| B | 0,06 |
| C | 0,09 |
| D | 0,06 |
| E | 0,5 |
| F | 0,3 |
| G | 0,02 |
| H | 0,05 |
| I | 0,02 |
| J | 0,004 |
| K | 0,02 |

Geprüfte Verbindungen

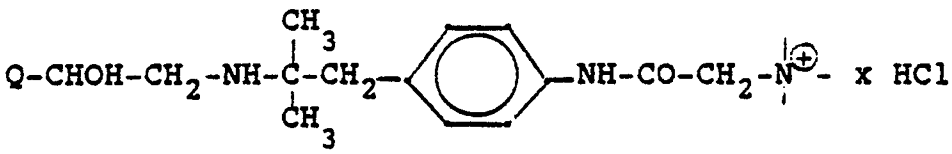

| Verbindung | Q | 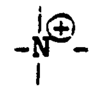 |
| --- | --- | --- |
| A: | 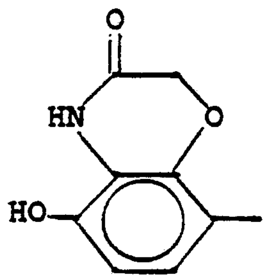 | $-N^{\oplus}(CH_3)_3Cl^{\ominus}$ |
| B: | 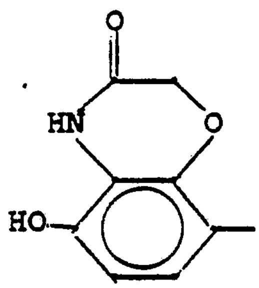 | 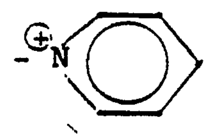 $Cl^{\ominus}$ |
| C: | 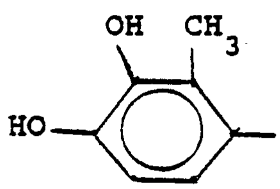 | $-N^{\oplus}(CH_3)_3Cl^{\ominus}$ |
| D: | 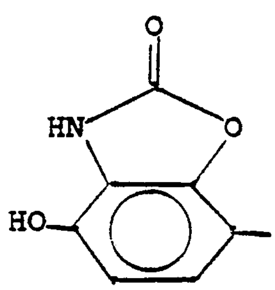 | $-^{\oplus}N(CH_3)_3Cl^{\ominus}$ |

| Verbindung | Q | $-\overset{|}{\underset{|}{N}}{}^{+}-$ |
| --- | --- | --- |
| E: | 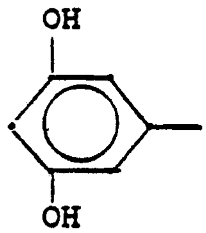 | $-^{\oplus}N(CH_3)_3Cl^{\ominus}$ |

Verbindungen der Formel

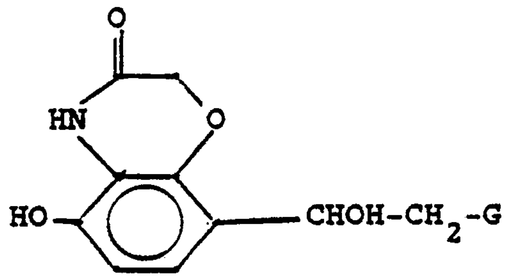

x HCl

| Verbindung | G |
|---|---|
| F: | 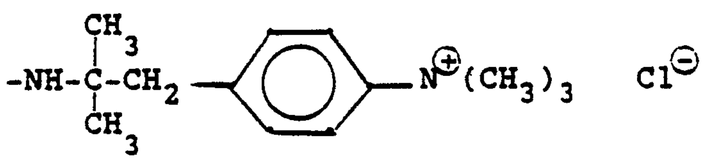 |
| G: | 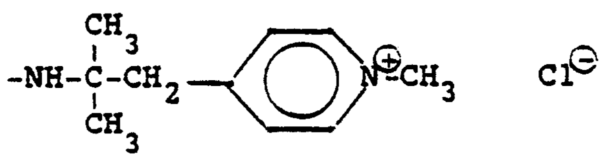 |
| H: | 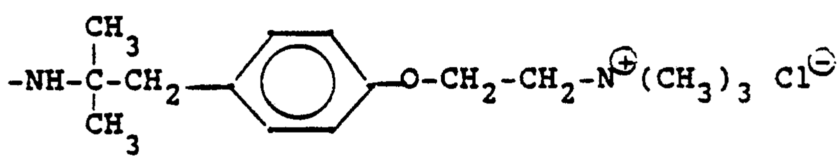 |
| I: | 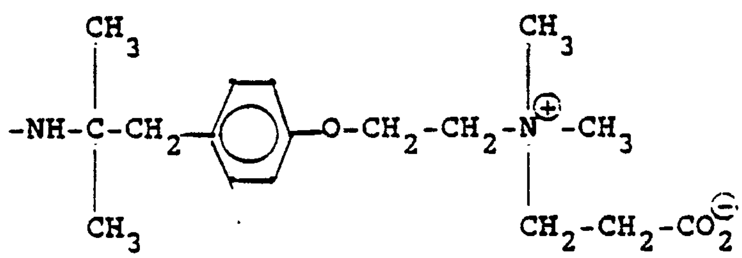 |
| J: | 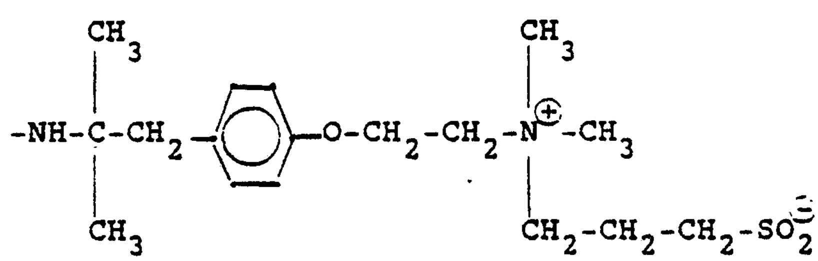 |

| Verbindung | G |
|---|---|
| K: | 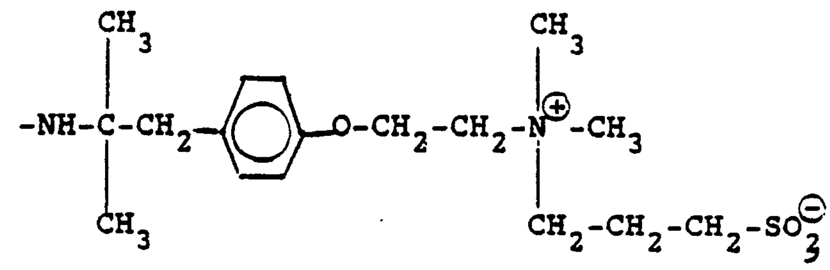 |

Zu Verfahren 1:

Beispiel 1

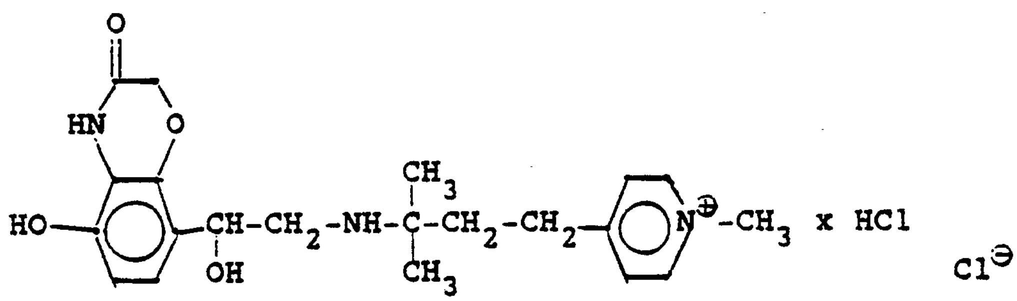

1,9 g 5′-Hydroxy-8′-{1-Hydroxy-2-[4-(4-Pyridyl)-2-methyl-2-butylamino]-ethyl}-2H-1,4-benzoxazin-3-(4H)-on-monohydrochlorid werden in einer Mischung von 3 ml Dimethylformamid/1 ml Wasser gelöst und mit 1,27 g Methyljodid zersetzt. Nach 12 Stunden wird die Lösung mit 5 ml Alkohol versetzt, mit konz. HCl angesäuert und mit Aceton verdünnt; die ausgefallenen Kristalle werden nach ~ 1 Stunde abgesaugt und durch Umfällen am Wasser, konz. Salzsäure und Alkohol erhält man 1,2 g der Verbindung.
Fp. 207-209° C , 56 % d.Th.

Die Ausgangsverbindung kann nach folgendem Verfahren hergestellt werden:

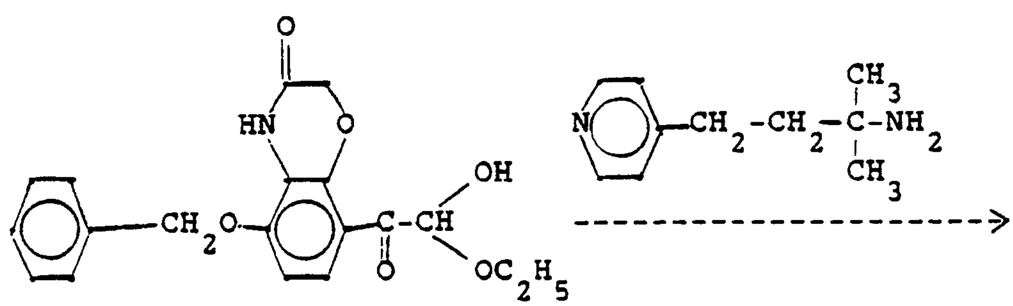

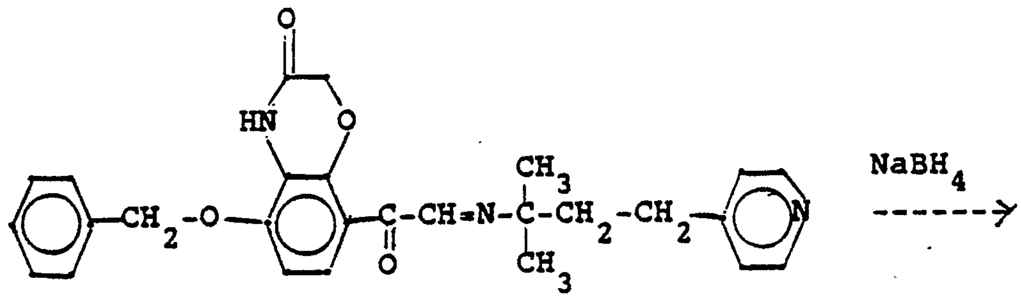


(Fp. 170-171°C)

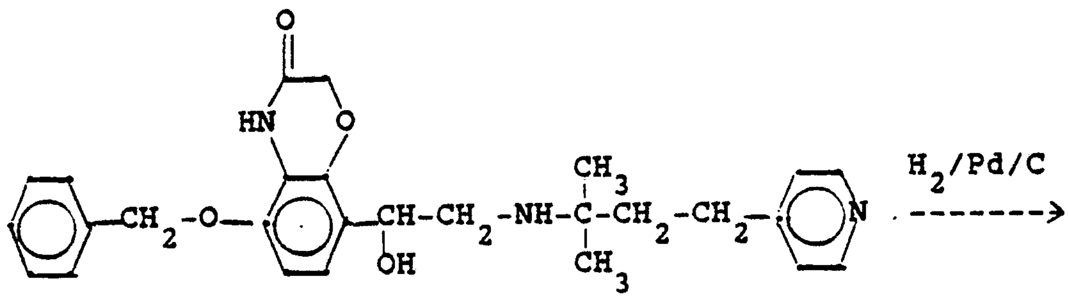


(Fp. 157-160°C)

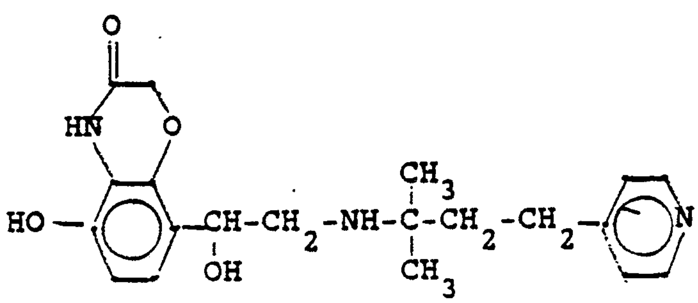

x HCl

(Fp. 175°C (Zers.)

Beispiel 2

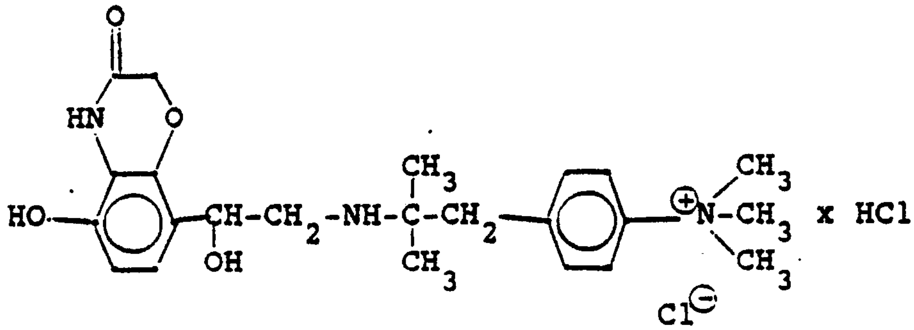

3,7 g 5′-Benzyloxy-8′-{1-hydroxy-2-[3-(4-dimethylaminophenyl)-2-methyl-2-propylamino]-ethyl}-2H-1,4-benzoxazin-3-(4H)-on-monohydrochlorid werden in 7,4 ml DMF mit 2,1 g Methyljodid versetzt und 12 Stunden reagieren lassen. Nach Verdünnen der Lösung mit Aceton erhält man das Ammoniumjodidhydrochlorid, welches durch Umsalzen mit Salzsäure oder über die Ammoniumhydroxydverbindung und Behandeln mit Salzsäure in die Ammonium-Chlor-hydrochlorid-Verbindung (Fp. 195-197° C) überführt wird.

3,7 g dieser Benzyloxyverbindung werden in 50 ml Methanol mit Palladiumkohle als Katalysator unter Normalbedingungen entbenzyliert und man erhält 2 g der Titelverbindung.
Fp. 187° C (Zers.); 6.28 % d.Th.).

Die Ausgangsverbindung kann nach folgendem Verfahren hergestellt werden:

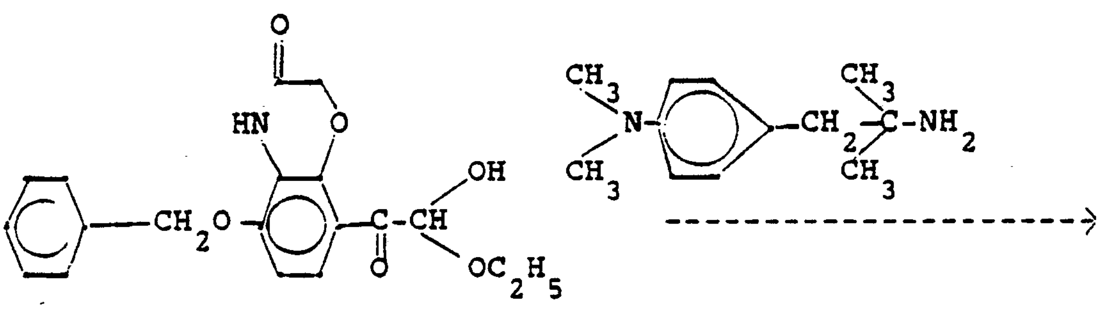

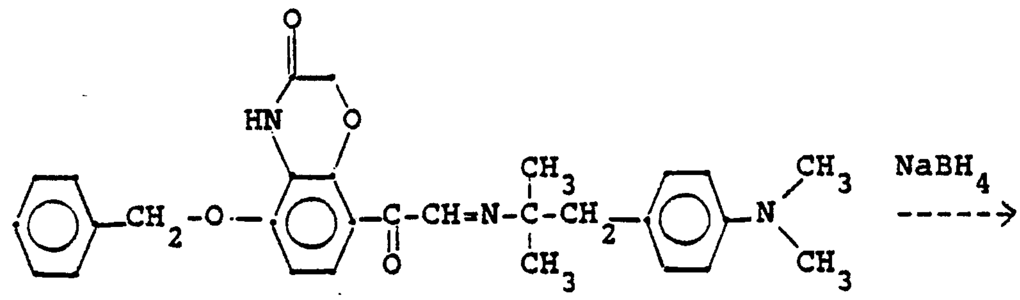


(Fp. 201-204°C)

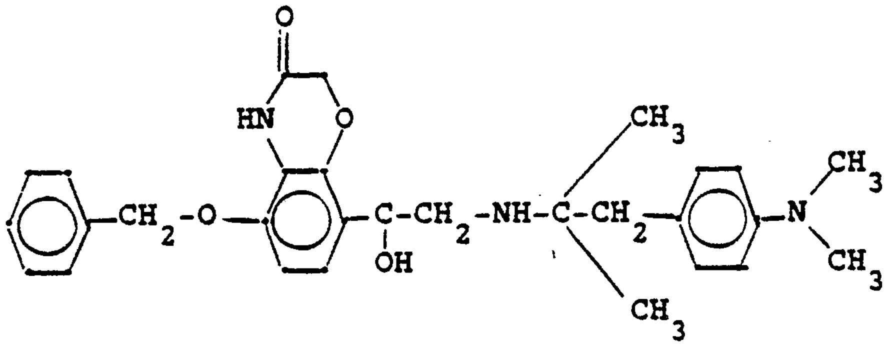

(Fp. 110-112°C, Fp. HCl Salz 232-235°C)

Beispiel 3

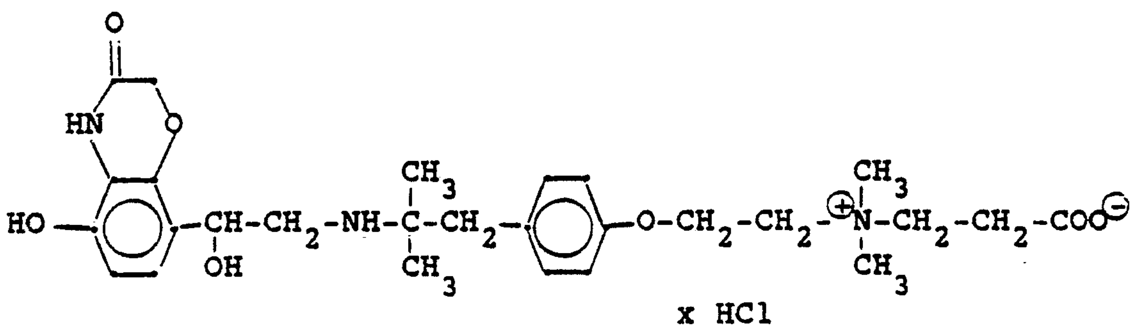

x HCl

3,2 g 5′-Benzyloxy-8′-{1-hydroxy-2-[3-(4-dimethylamino-ethoxyphenyl)-2-methyl-2-propylamino]-ethyl}-2H-1,4-benzoxazin-2-(4H)-on-monohydrochlorid werden in 6 ml Aceton mit 0,41 g β-Propiolacton versetzt und 12 Stunden bei Raumtemperatur reagieren lassen. Nach dem Verdünnen mit Aceton werden die ausgefallenen Kristalle abgesaugt und man erhält 2,4 g der Verbindung (Fp. 123-126° C).

2,3 g der Benzyloxyverbindung werden in 50 ml Methanol unter Zusatz von Palladiumkohle entbenzyliert und man erhält 1,7 g der Titelverbindung.

Fp. 173-175° C, 94 % d.Th.).

Die Ausgangsverbindung kann nach folgendem Verfahren hergestellt werden:

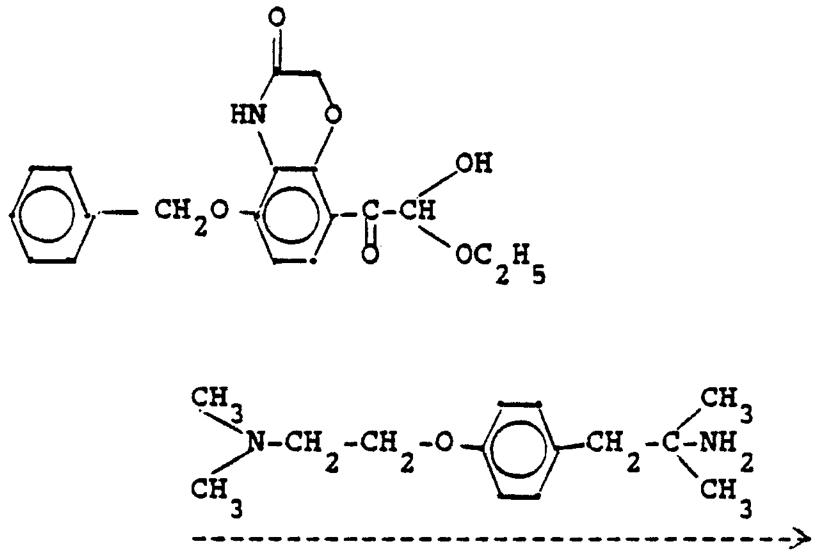

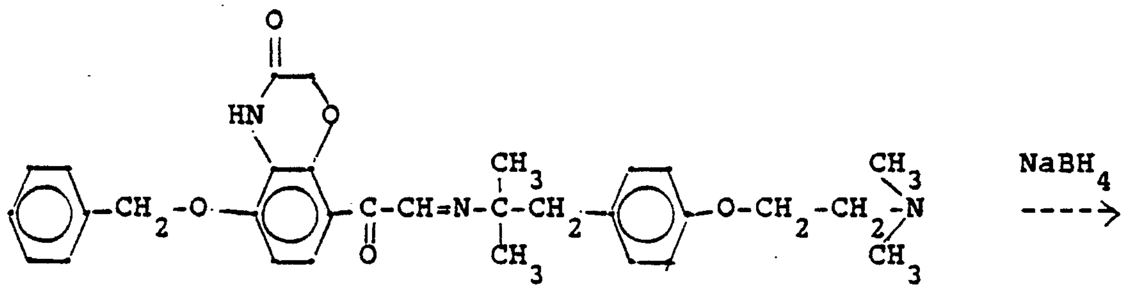


(Fp. 153-155°C)

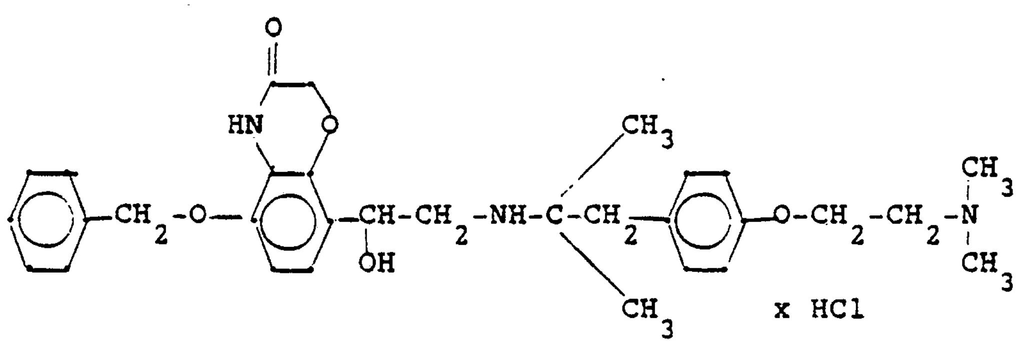

(Fp. 176-178°C)

Entsprechend den angegebenen Beispielen werden synthetisiert:

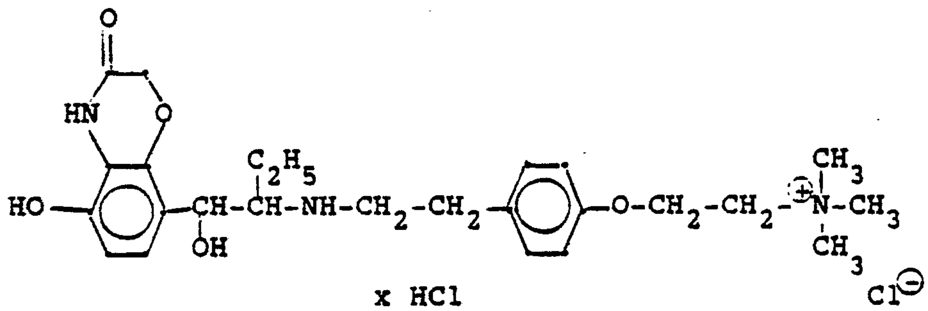

x HCl

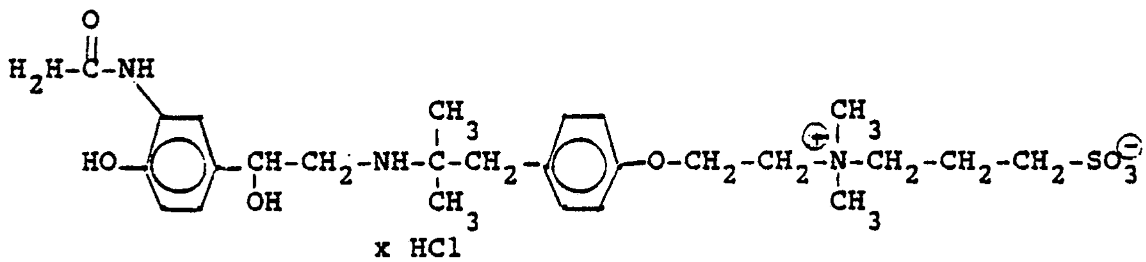

x HCl

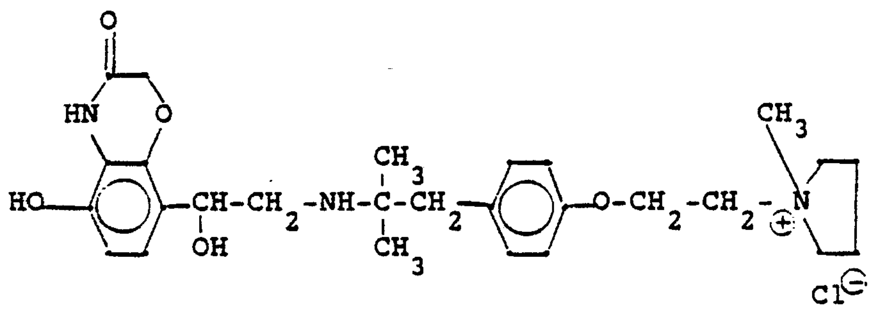

x HCl

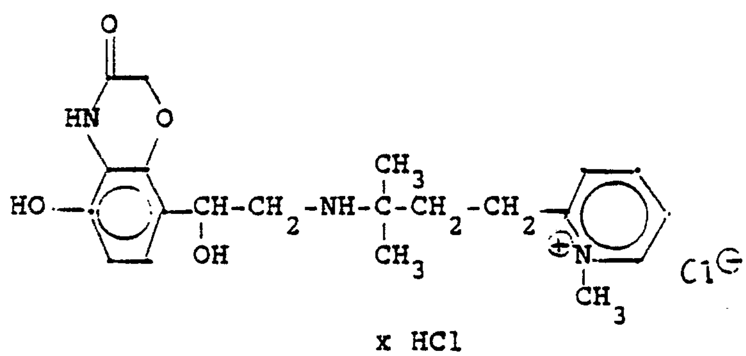

x HCl

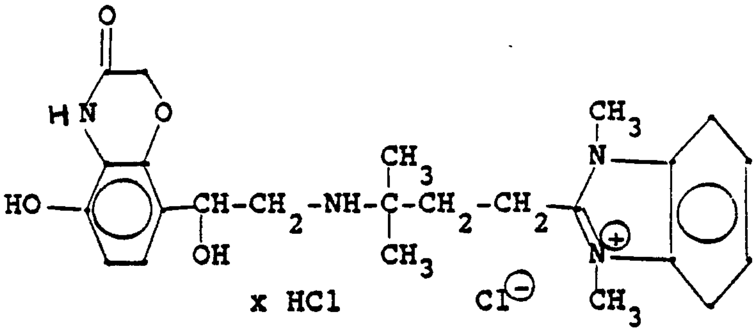

x HCl

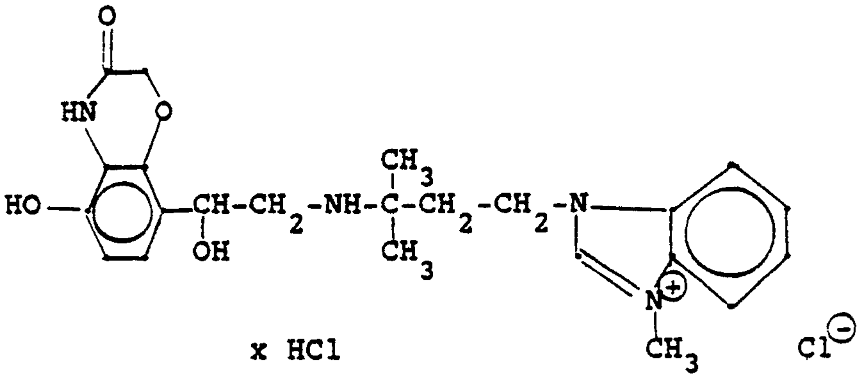

x HCl

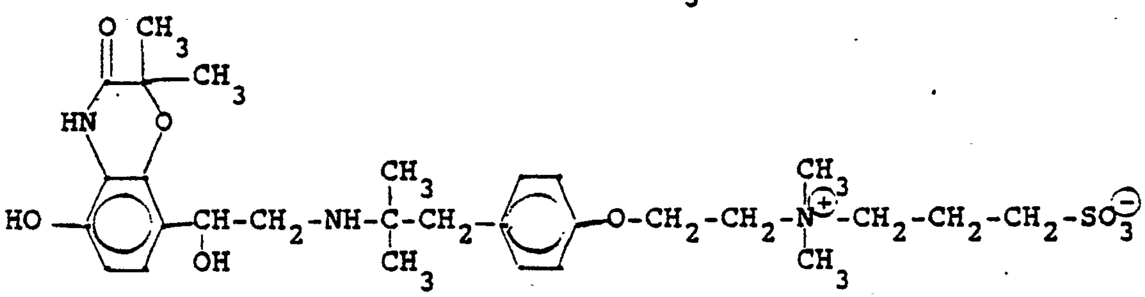

x HCl

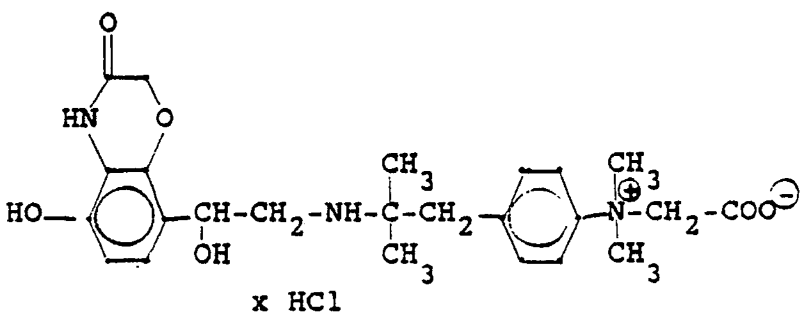

x HCl

(Fp. 175°C)

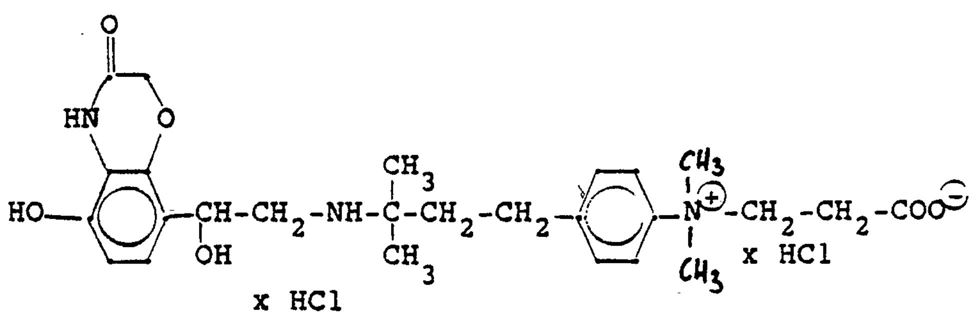

x HCl

x HCl

(Fp. 191-193°C)

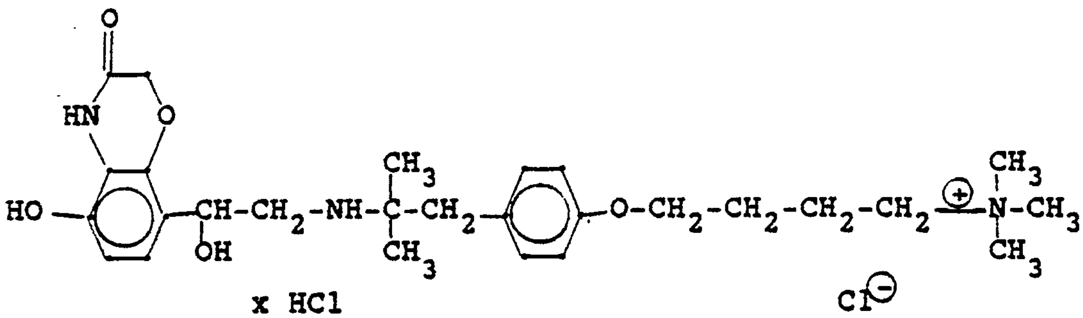

x HCl

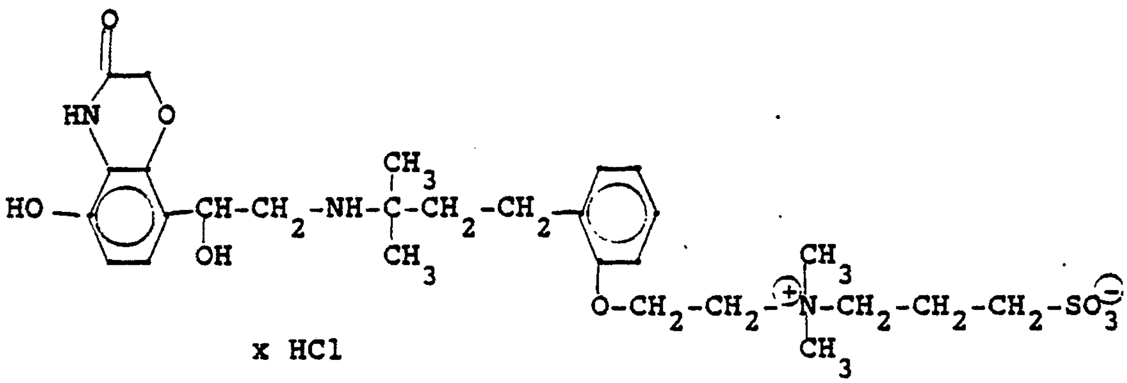

x HCl

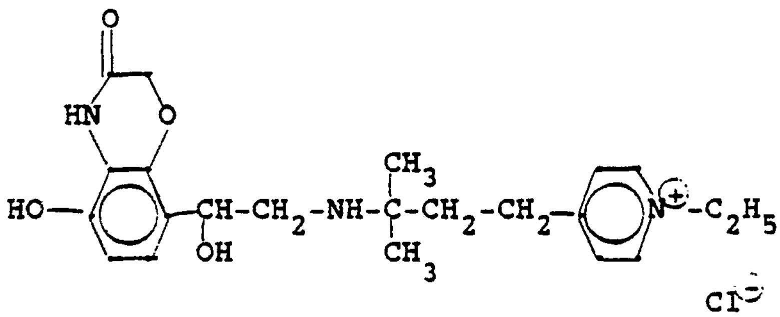

x HCl

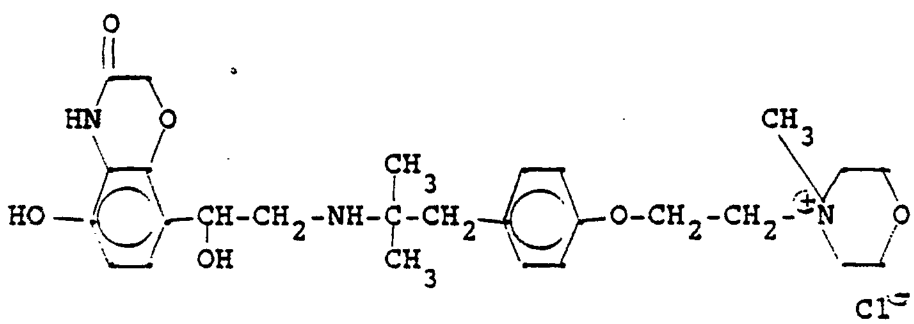

x HCl

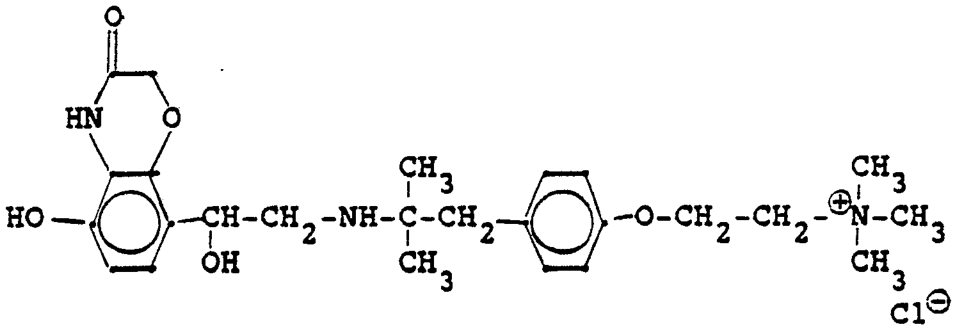

x HCl (Fp. 235°C)

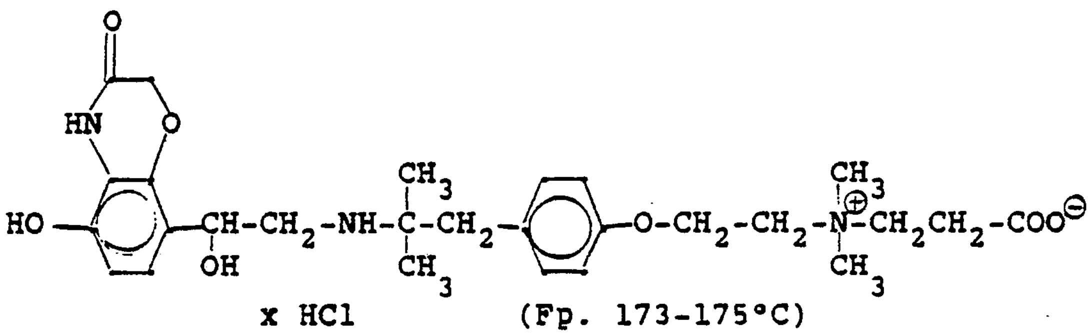

x HCl (Fp. 173-175°C)

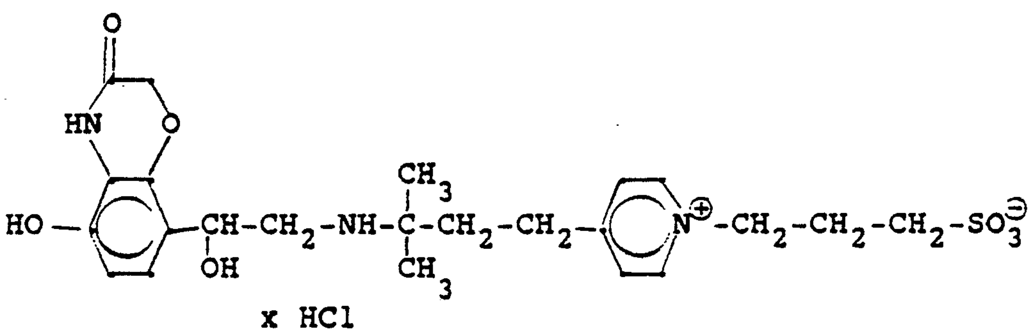

x HCl

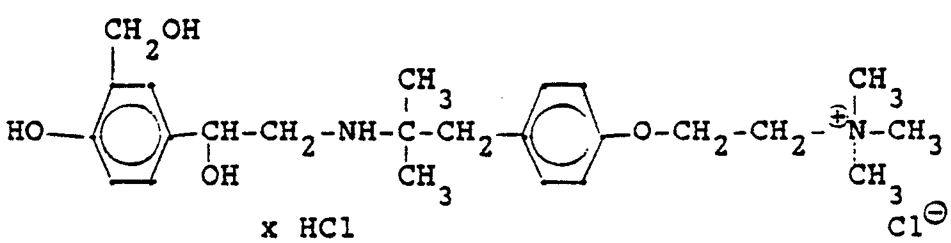

x HCl

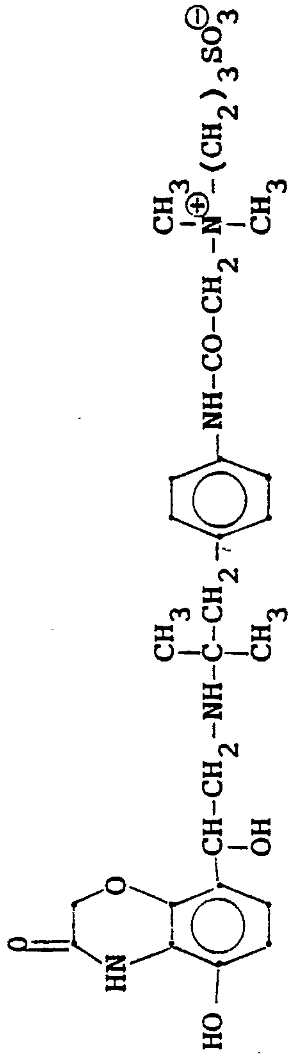

x HCl x $H_2O$ (Fp. 250-255°C)

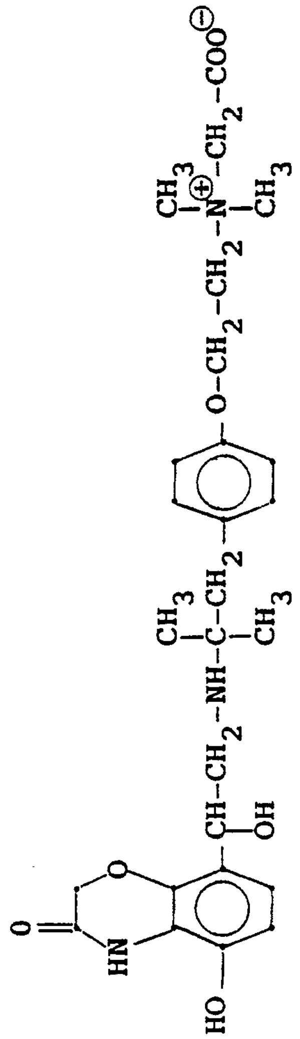

x HCl x $H_2O$ (Fp. 214-216°C)

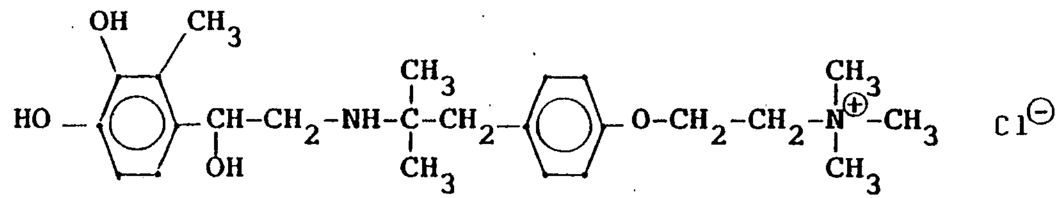

x HCl

(Fp. 158-162°C)

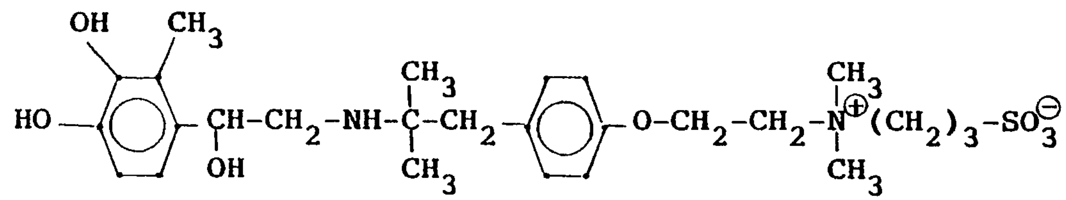

x

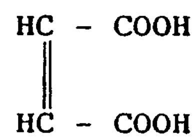

(Fp. 220-223°C)

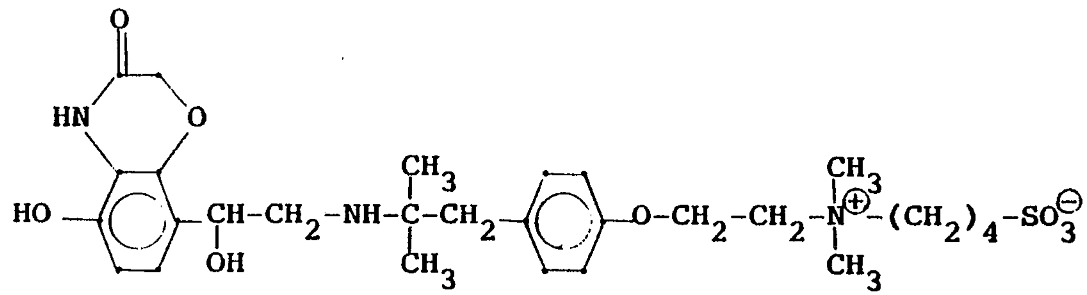

x HCl x 1/2 $H_2O$ (Fp. 231-234°C)

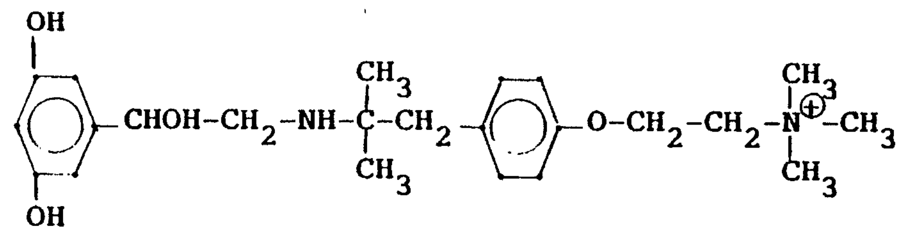

x 1/2 $H_2SO_4$ x 1/2 $SO_4^{2-}$ x $H_2O$

(Fp. 263-265°C)

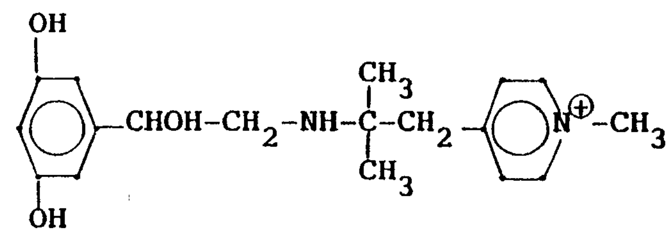

x 1/2 $H_2SO_4$ x 1/2 $SO_4^{2-}$ x $H_2O$
(Fp. > 270°C)

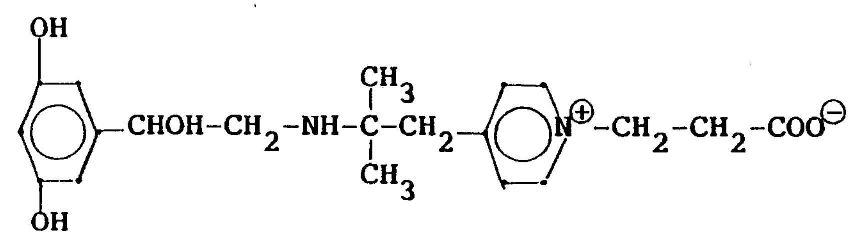

x HCl x $2H_2O$
(Fp. 171-174°C)

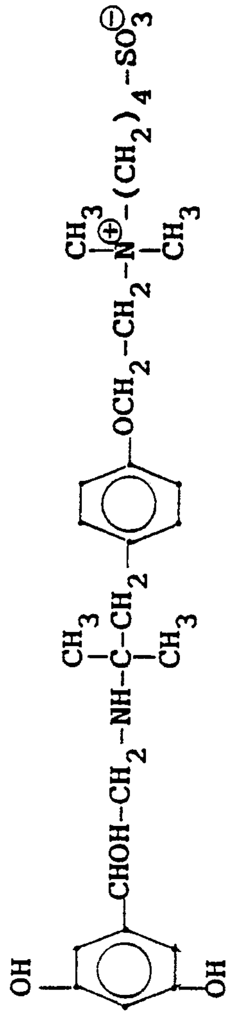

x 1/2 $H_2SO_4$ x $H_2O$
(Fp. 234-236°C)

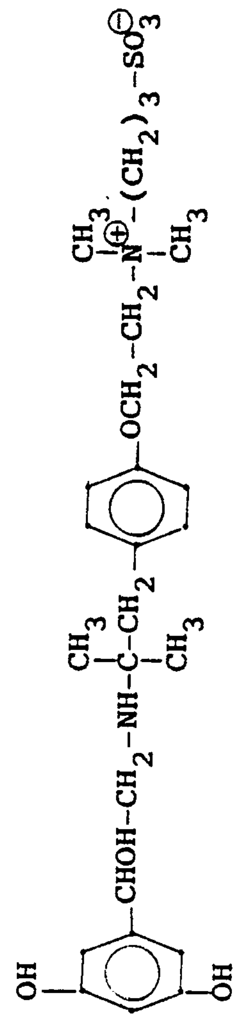

x HCl
(Fp. 231-234°C)

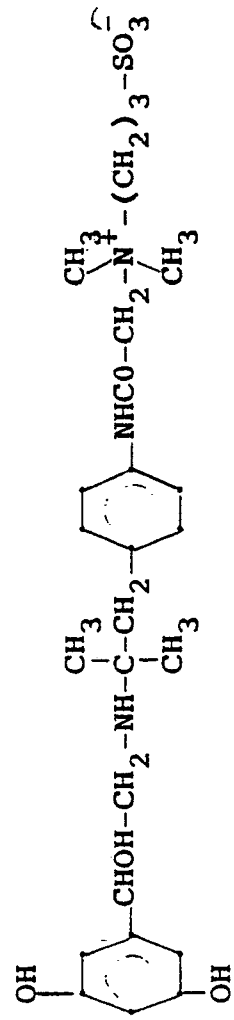

x HCl x 1,5 $H_2O$
(Fp. 210°C)

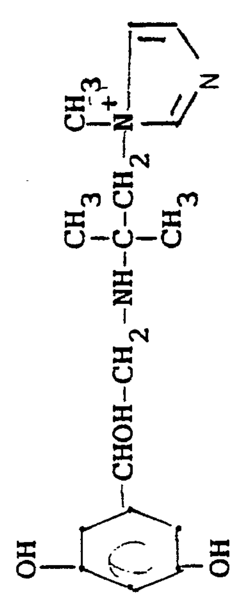

x HCl x $2H_2O$

(Fp. 95-100°C)

Beispiel 4

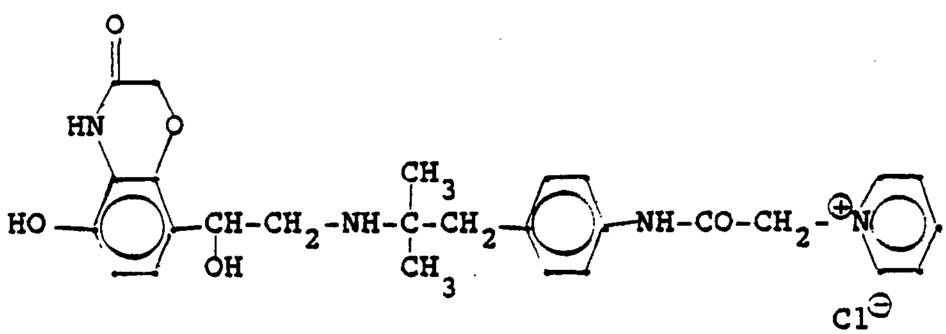

x HCl

4,4 g 5′-Hydroxy-8′-{1-hydroxy-2-[3-(4-chloracetaminophenyl)-2-methyl-2-propylamino]-ethyl}-2H-1,4-benzoxazin-3-(4H)-on-hydrochlorid, 6 ml Pyridin und 25 ml Methanol werden 6 Stunden refluxiert, nach dem Abdestillieren des Methanols und Pyridins wird der ölige Rückstand in Alkohol gelöst und man erhält 3,8 g der Titelverbindung.

(Fp. 190-192° C; 77,5 % d. Th.).

Die Ausgangsverbindung kann nach folgenden Verfahren hergestellt werden:

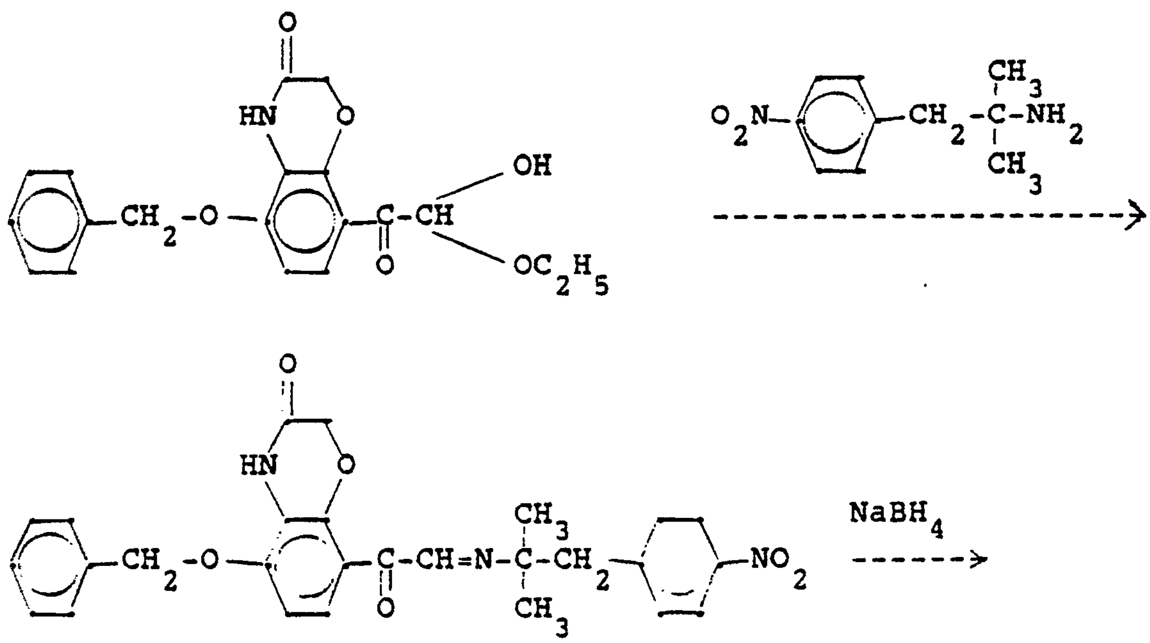


(Fp. 151-154°C)

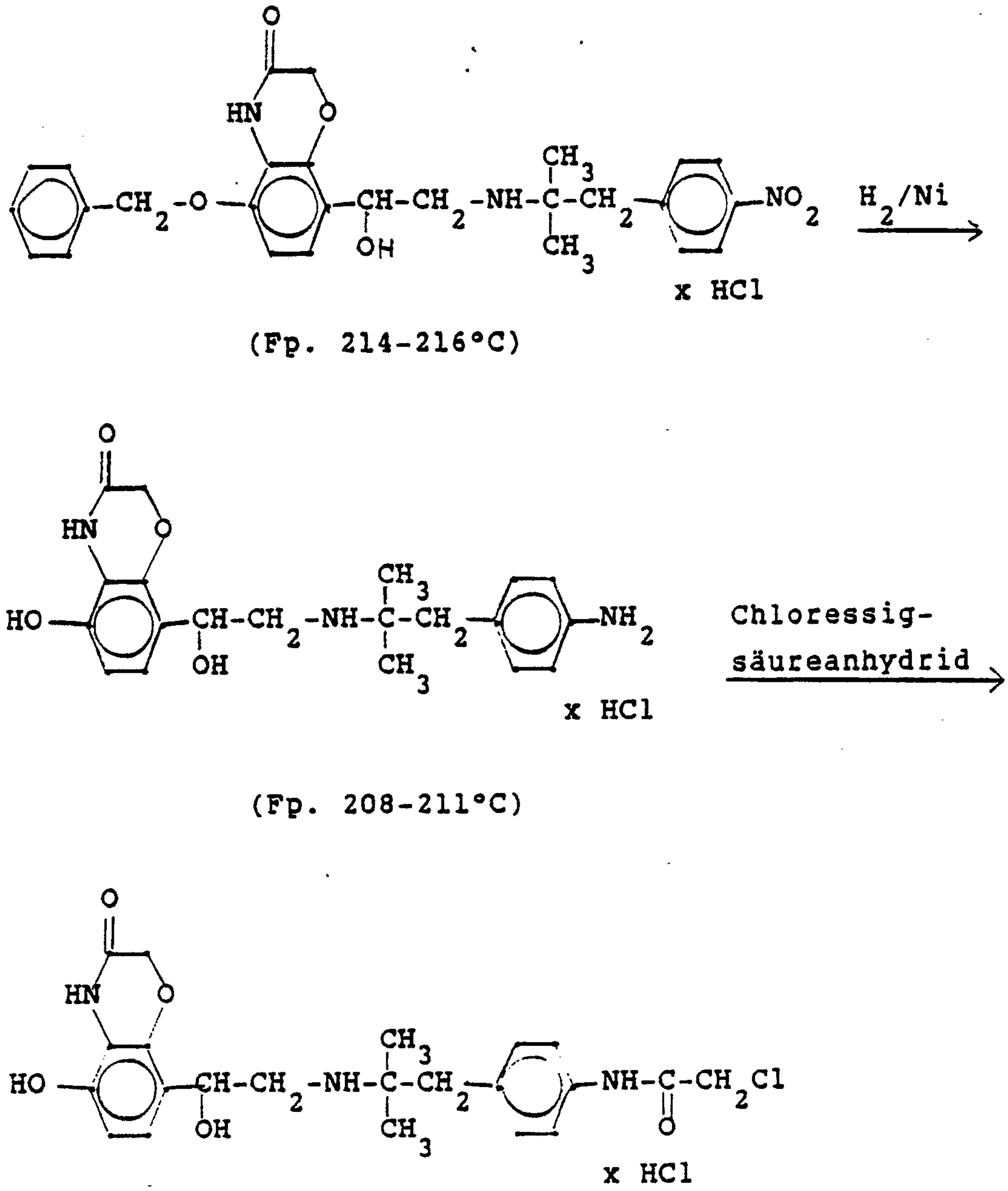


(Fp. 214-216°C)

(Fp. 208-211°C)

(Fp. 160°C Zers.)

Beispiel 5

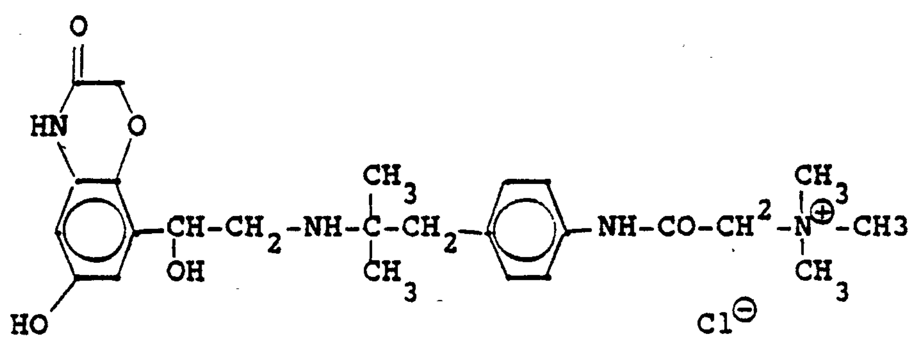

2,7 g 6′-Hydroxy-8′-{1-hydroxy-2-[3-(4-chloracetaminophenyl)-2-methyl-2-propylamino]-ethyl}-2H- 1,4-benzoxazin-3-(4H)-on-hydrochlorid, 25 ml Methanol und 3 ml 30%ige Trimethylaminlösung werden 12 Stunden bei Raumtemperatur gerührt, konzentriert und das erhaltene Öl in Alkohol gelöst. Nach 1 Stunde werden die ausgefallenen Kristalle abgesaugt und man erhält 2 g der Titelverbindung. (Fp. 203-206° C, 65% d.Th.)

Die Ausgangsverbindung kann nach folgendem Verfahren hergestellt werden:

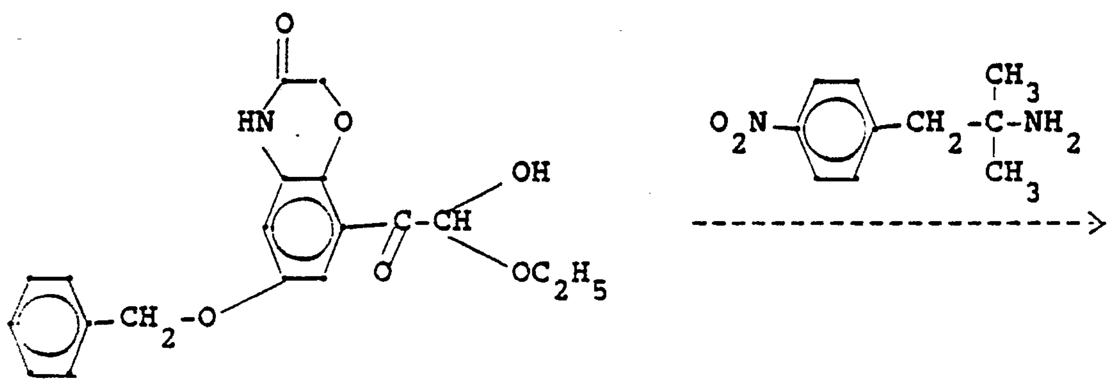

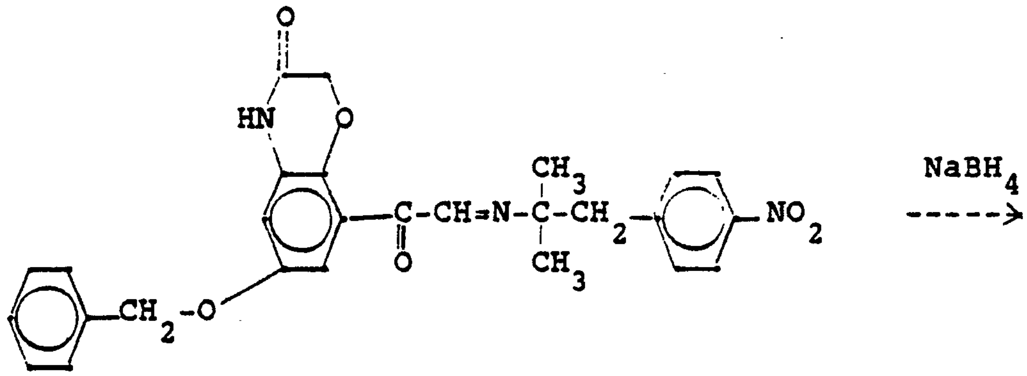


(Fp. 140-142°C)

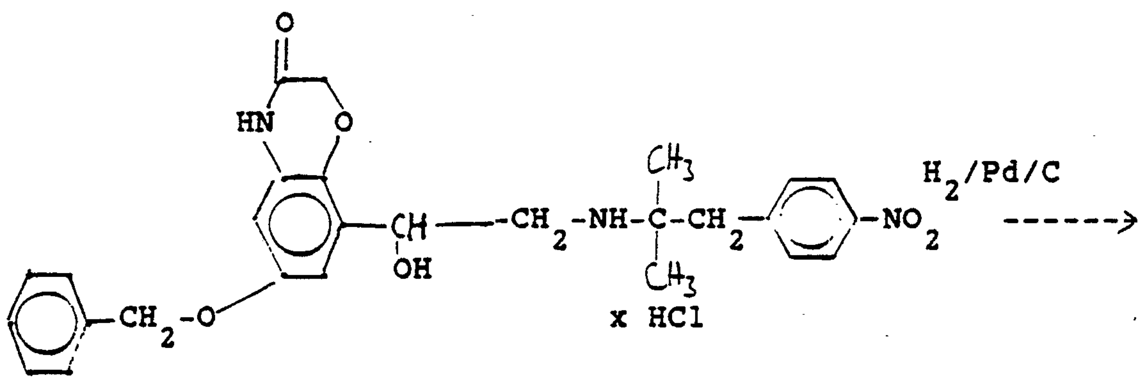


(Fp. 232-234°C)

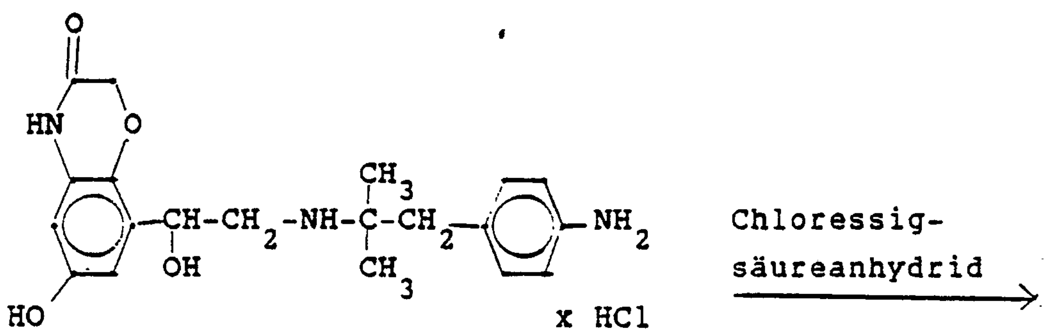


(Fp. 266-268°C)

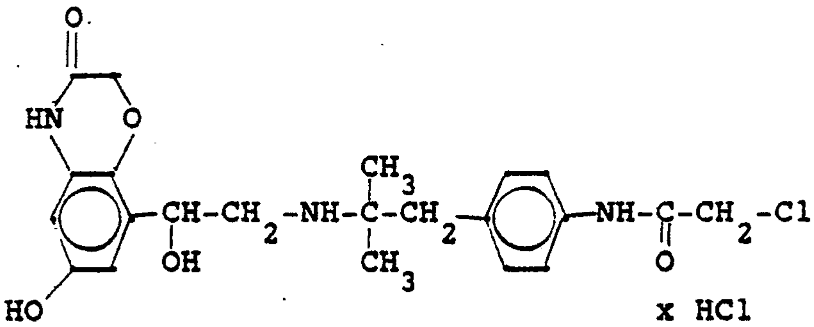

x HCl

(Fp. 185-189°C)

In analoger Weise werden folgende Verbindungen hergestellt:

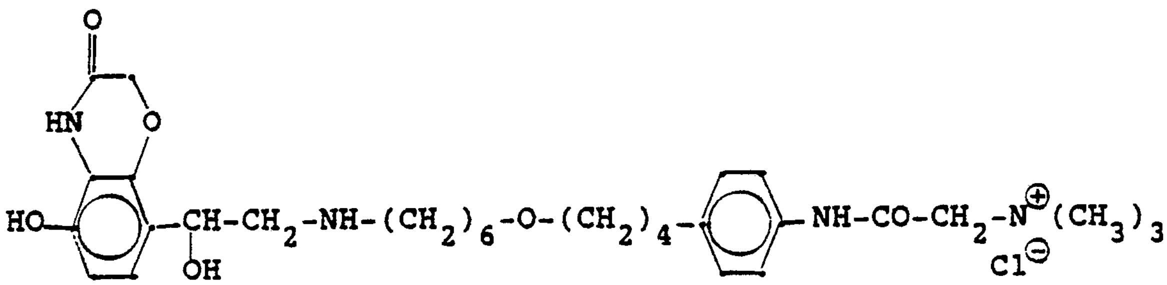

x HCl

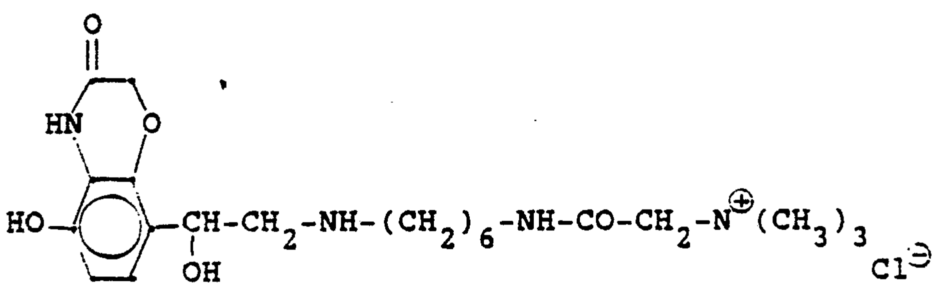

x HCl

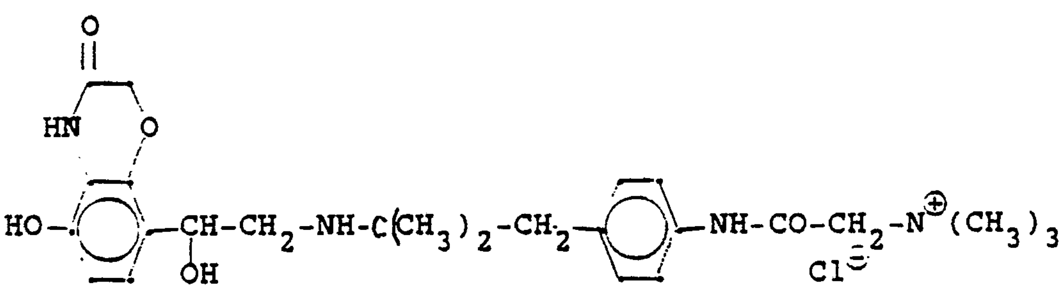

x HCl

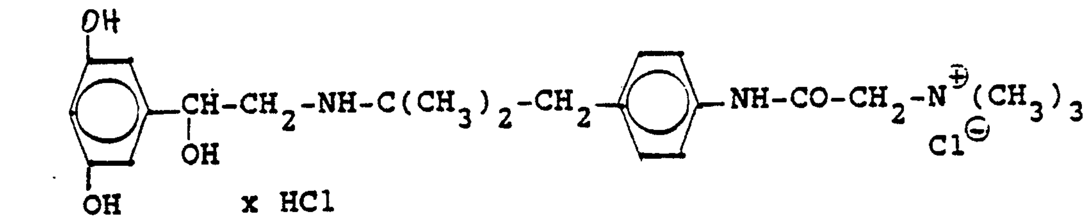

x HCl

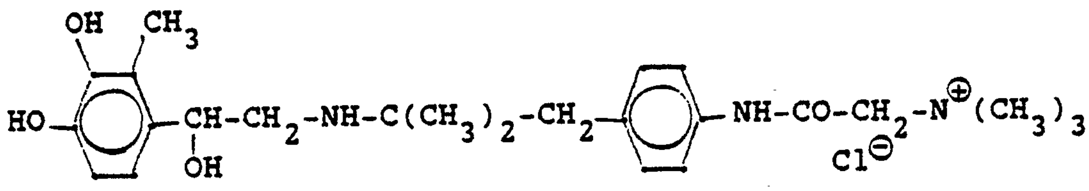

x HCl

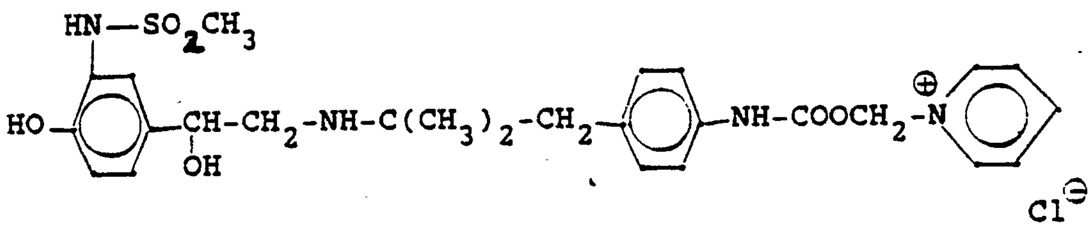

x HCl

(Fp. 164-167°C)

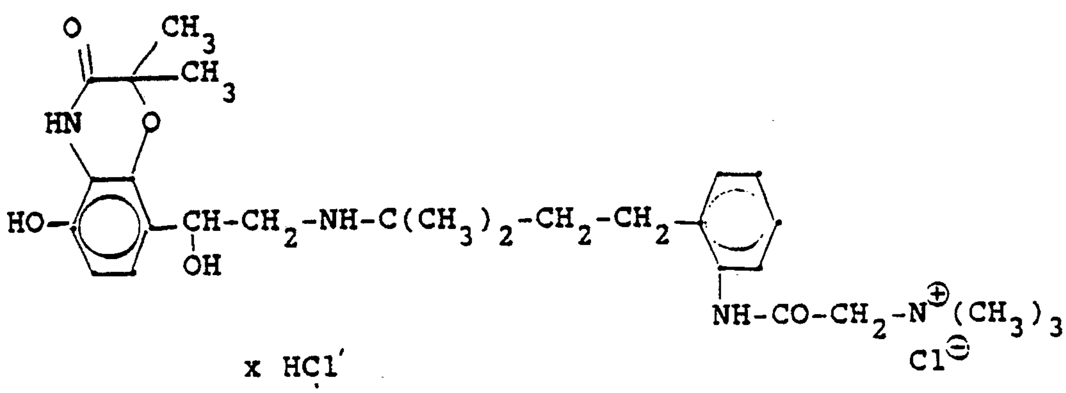

x HCl

Zu Verfahren 3

Beispiel 6

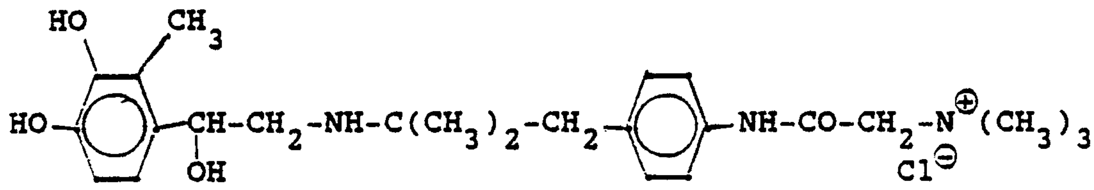

4,2 g der Dibenzyloxyverbindung (siehe unten) werden mit Wasserstoff in Methanol mit Palladiumkohle als Katalysator unter Normalbedingungen entbenzyliert und man erhält 2,5 g der Titelverbindung. (81 % d.Th.).

Die Ausgangsverbindung kann nach folgendem Verfahren hergestellt werden:

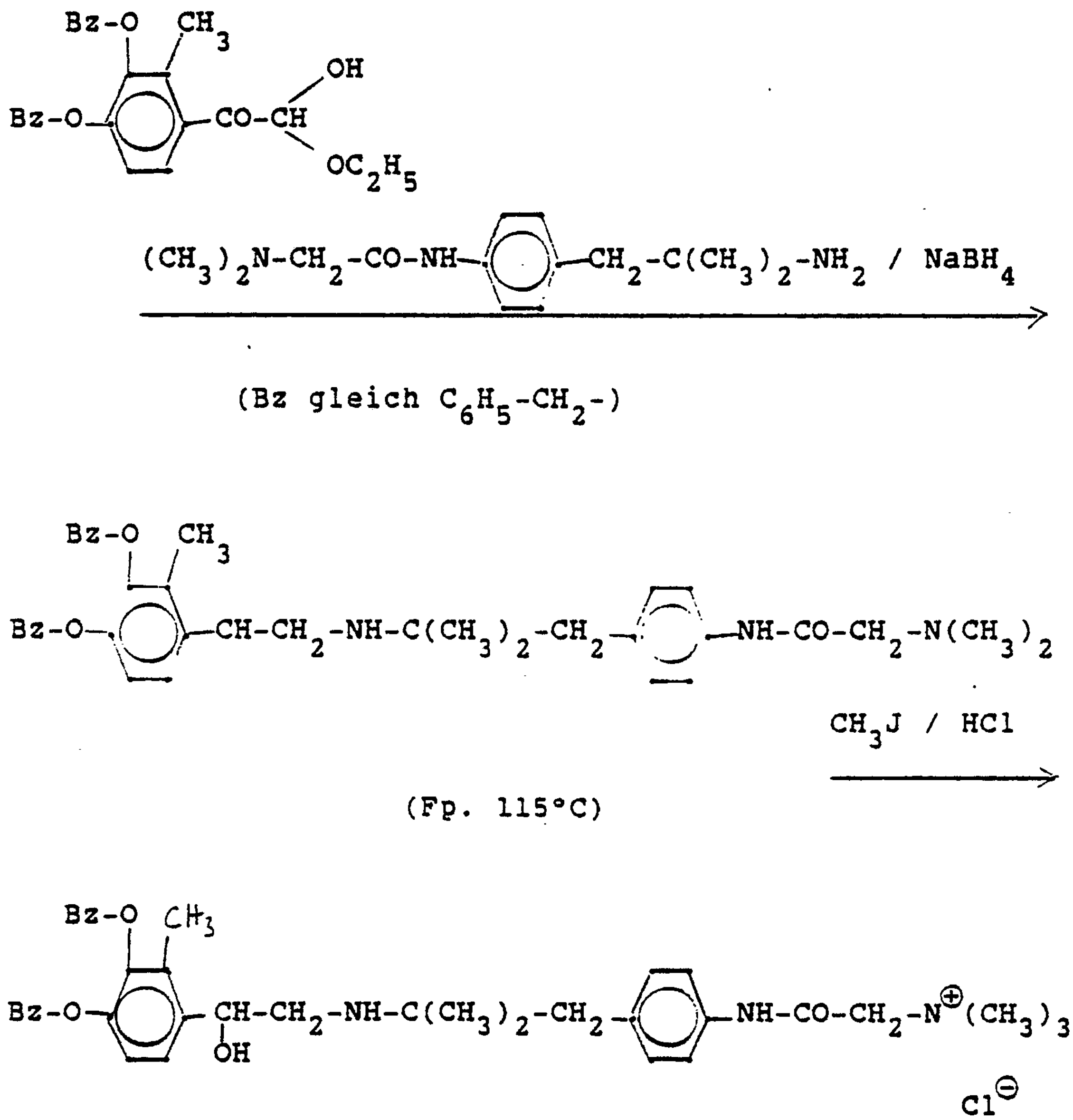


(Fp. 115°C)

(Fp. 210-212°C)

Beispiel 7

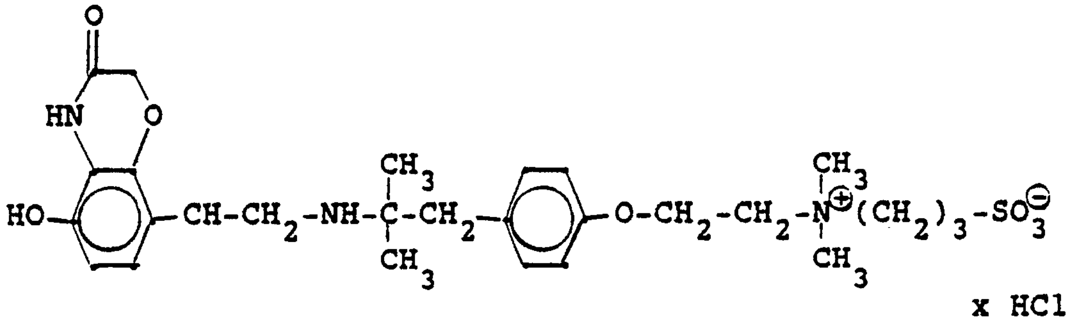

x HCl

3,5 g Benzyloxyverbindung (siehe unten) werden in 50 ml Methanol mit Wasserstoff unter Zusatz von 0,5 g Palladiumkohle (5%ig) als Katalysator unter Normalbedingungen entbenzyliert. Nach beendeter Aufnahme wird der Katalysator abgesaugt, das Methanol unter vermindertem Druck am Rotavapor abdestilliert und der Rückstand in ca. 90%igem Alkohol gelöst. Nach dem Animpfen werden die ausgefallenen Kristalle abgesaugt, mit Alkohol gewaschen und getrocknet. Man erhält 2,9 g der Titelverbindung, (Fp. 240 °C; 93 % d.Th.)

Die Ausgangsverbindung kann nach folgendem Verfahren hergestellt werden (Bz gleich Benzyl)

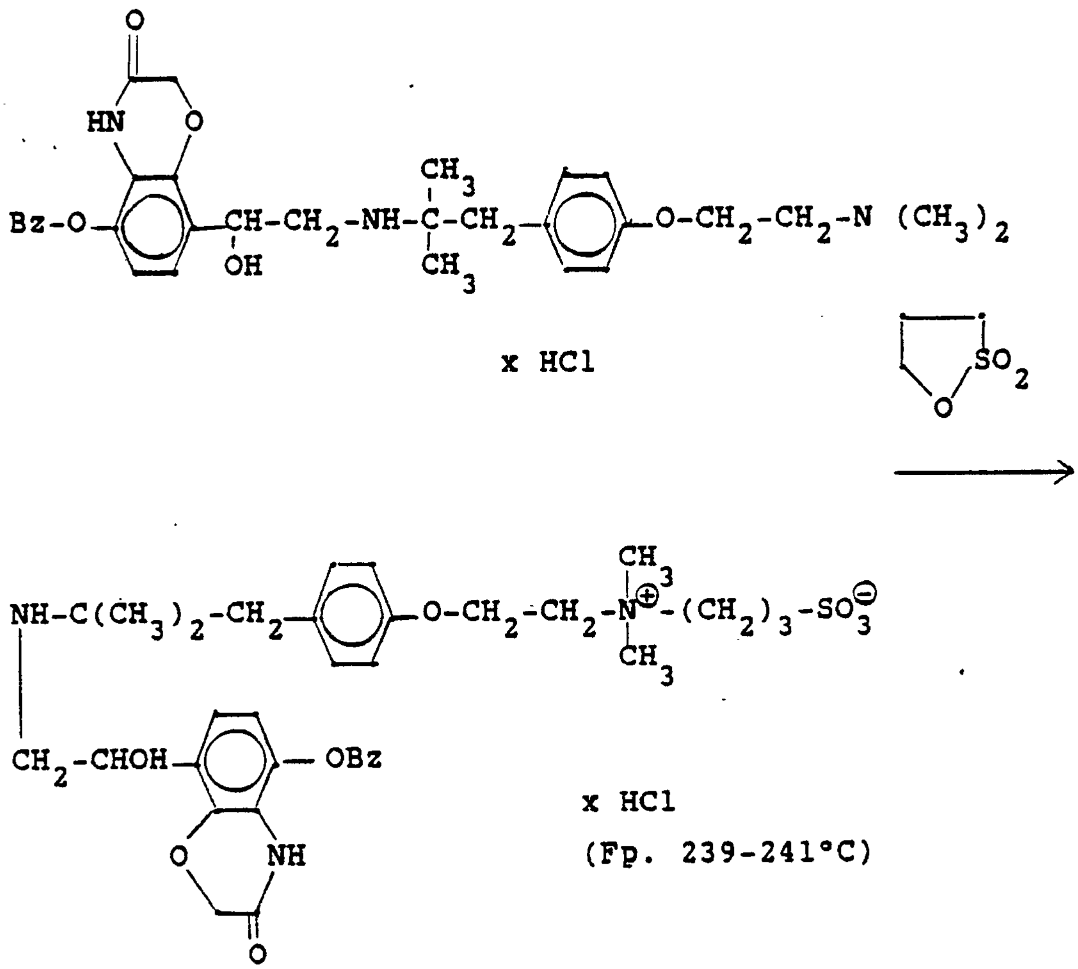


Beispiel 8

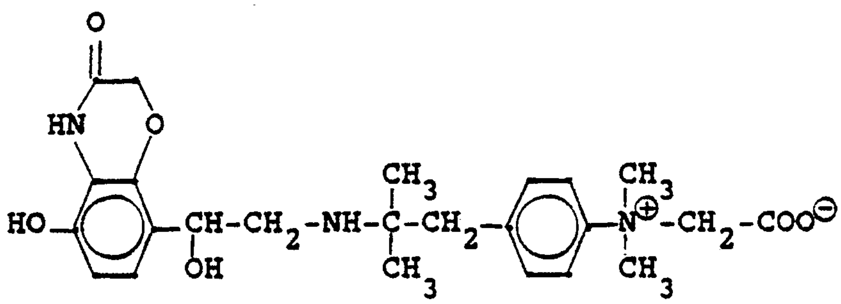

2,4 g Benzyloxyverbindung werden in 50 ml Methanol und 10 ml Wasser mit Wasserstoff unter Zusatz von 0,5 g Palladiumkohle als Katalysator unter Normalbedingungen entbenzyliert. Nach beendeter Aufnahme wird der Katalysator abgesaugt, das Methanol unter vermindertem Druck am Rotavapor abdestilliert und der Rückstand mit Alkohol verrieben. Die ausgefallenen Kristalle werden abgesaugt und einmal mit Wasser/Alkohol umgefällt. Man erhält 1,6 g der Titelverbindung
(Fp. 175 °C Zers., 71%dTh.)

Die Ausgangsverbindungen werden nach folgendem Verfahren hergestellt:

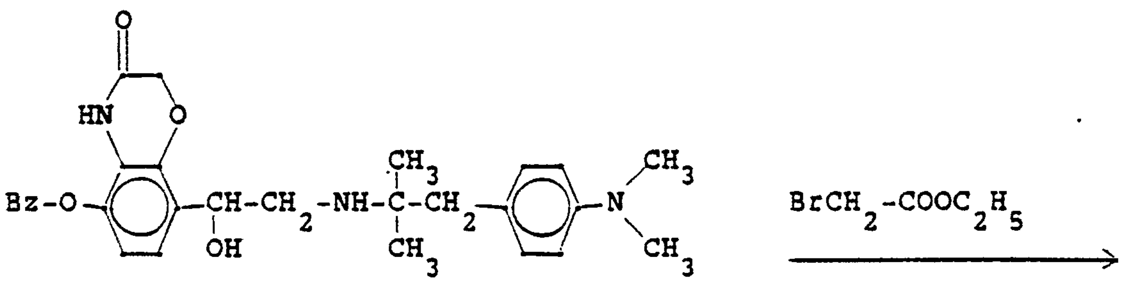


x HCl

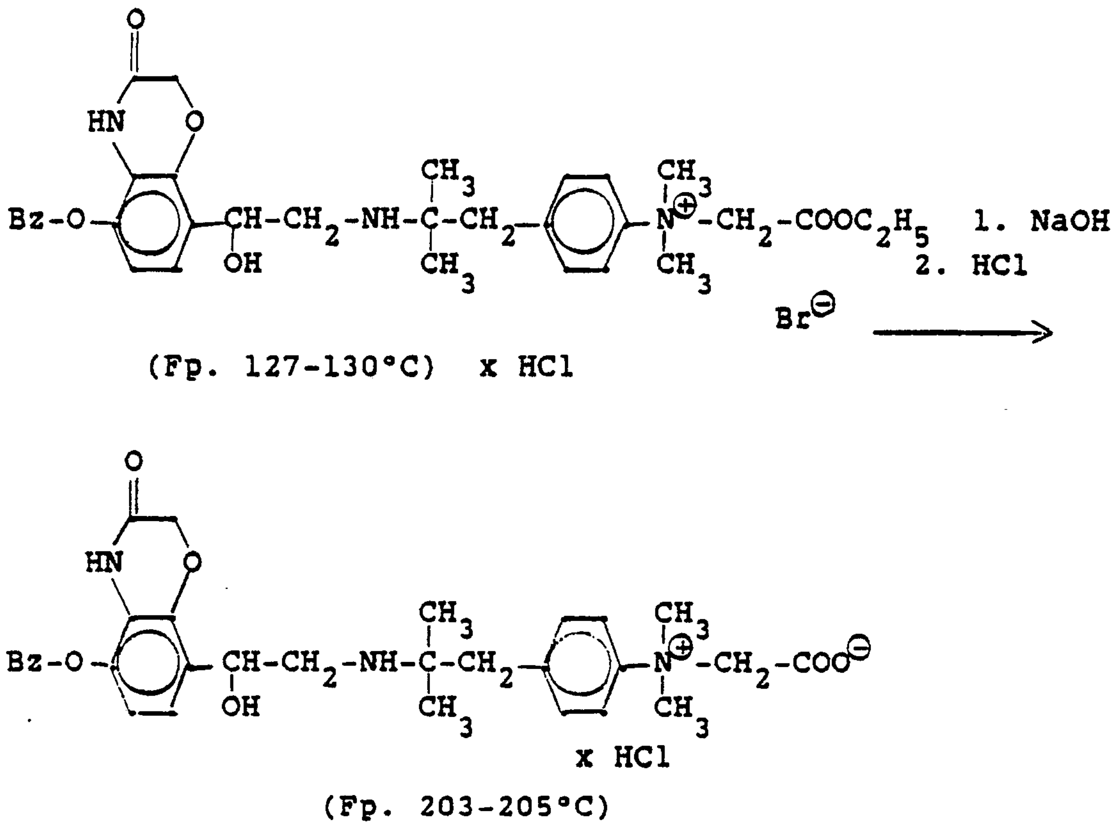


Entsprechend den angegebenen Beispielen werden synthetisiert:

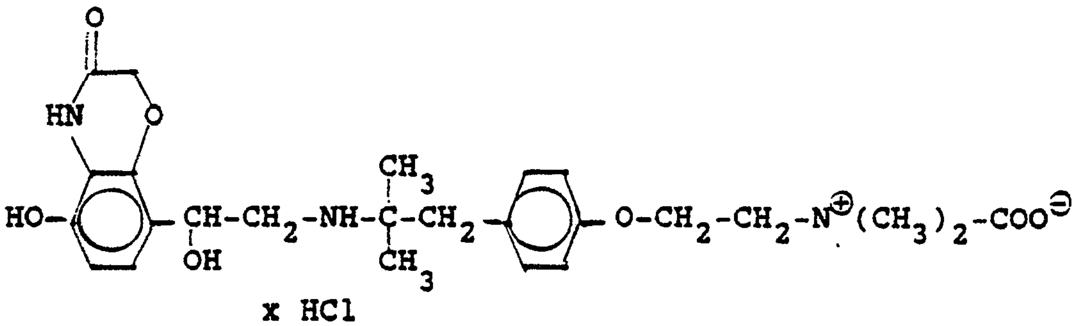

x HCl

(Fp. 173-175°C)

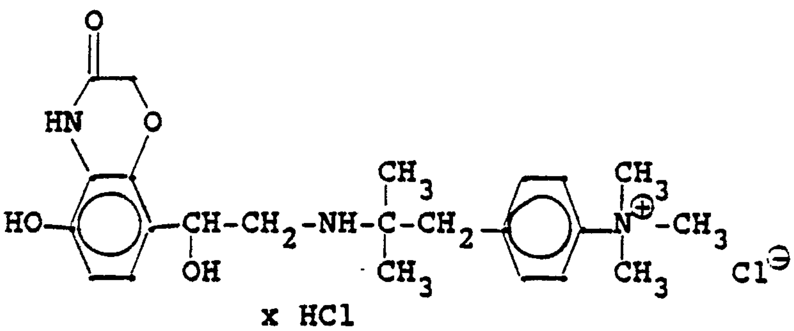

x HCl

(Fp. 187°C)

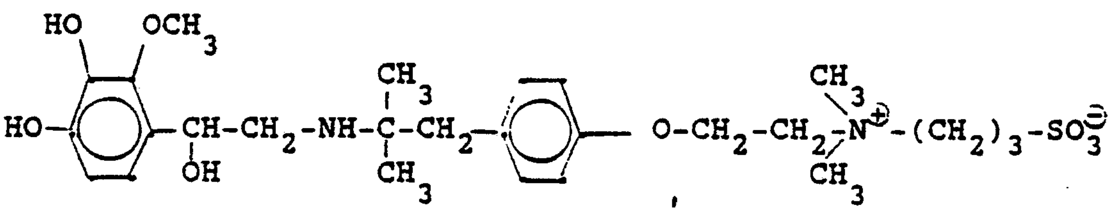

,

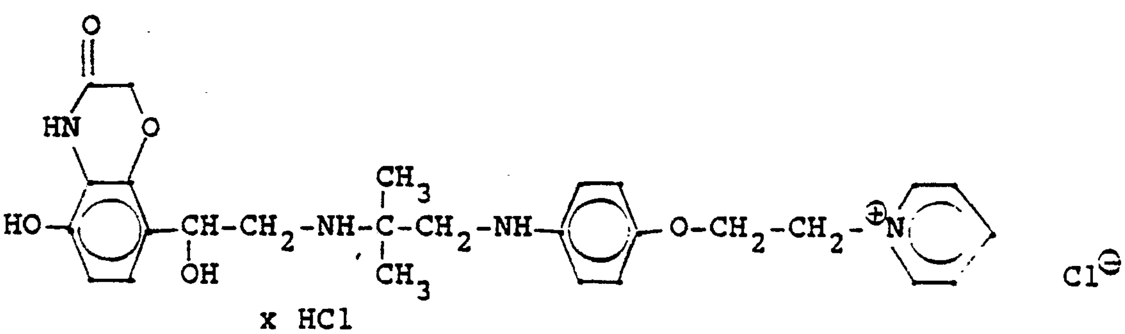

x HCl

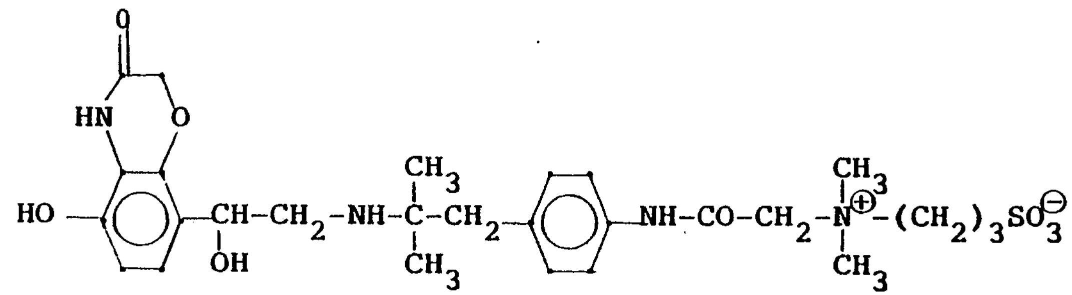

x HCl x $H_2O$ (Fp. 250-255°C)

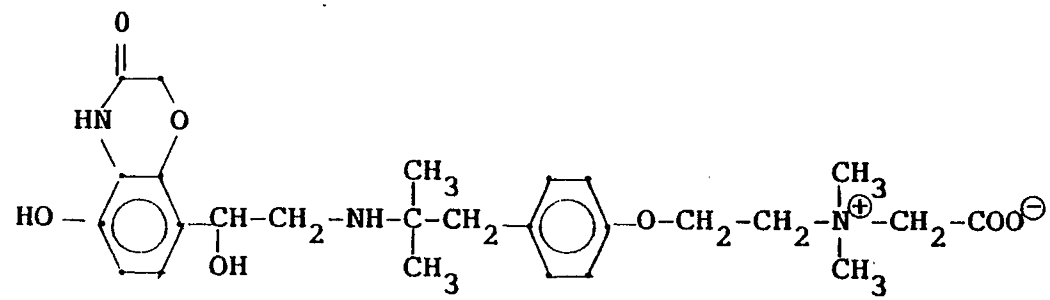

x HCl x $H_2O$ (Fp. 214-216°C)

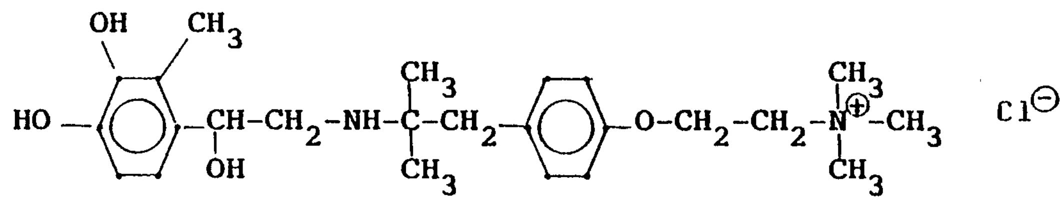

x HCl (Fp. 158-162°C)

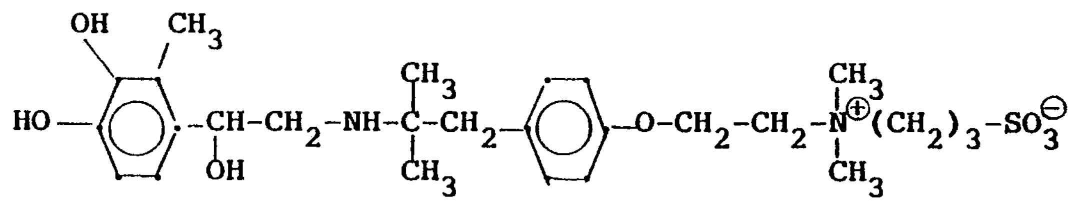

x 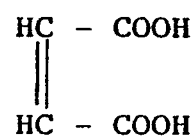

(Fp. 220-223°C)

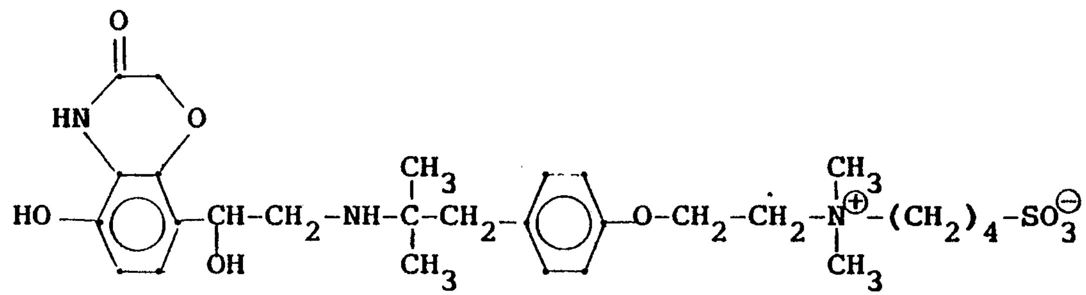

x HCl x $H_2O$ (Fp. 231-234°C)

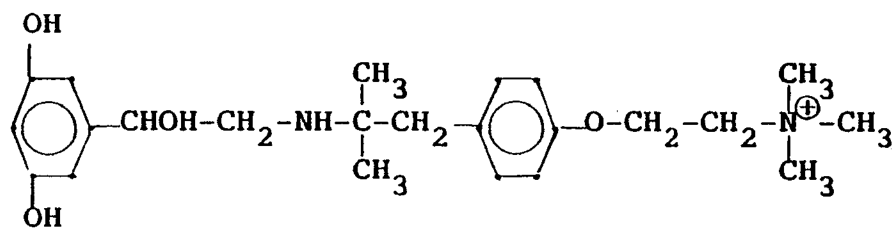

x 1/2 $H_2SO_4$ x 1/2 $SO_4^{2\ominus}$ x $H_2O$

(Fp. 263-265°C)

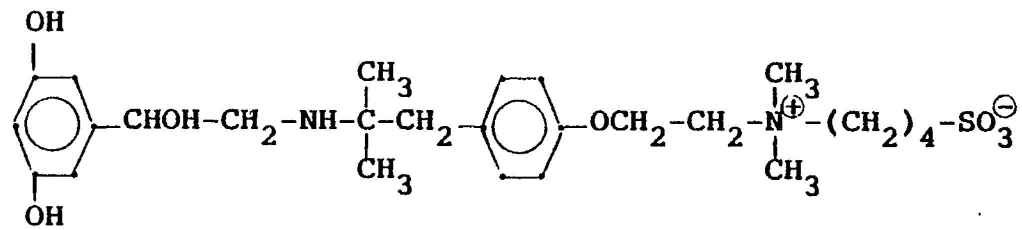

x 1/2 $H_2SO_4$ x $H_2O$
(Fp. 234-236°C)

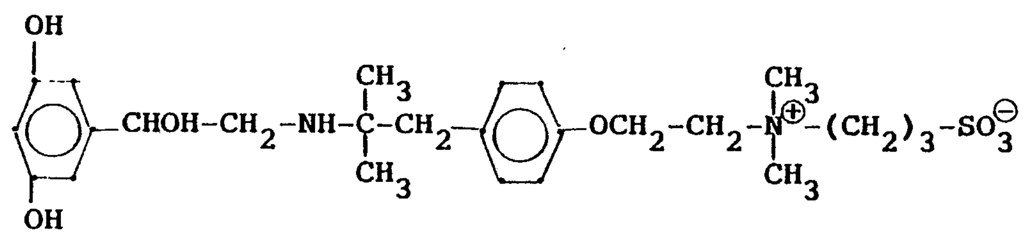

x HCl
(Fp. 231-234°C)

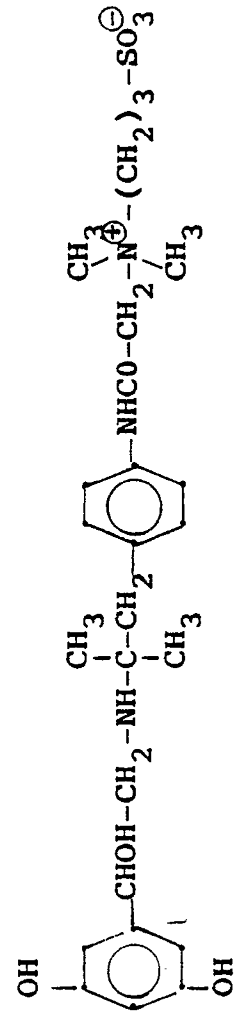

x HCl x 1,5 $H_2O$
(Fp. 210°C

## Ansprüche

1. Verbindungen der Formel

$$Q - \underset{\substack{|\\OH}}{CH} - \overset{\substack{R_4\\|}}{CH} - NH - \overset{\substack{R_5\\|}}{\underset{\substack{|\\R_6}}{C}} - (CH_2)_n - R \qquad (I),$$

in der

Q: den substituierten Phenylrest broncholytisch wirksamer Verbindungen;

R: einen Rest, insbesondere einen Alkoxy-, Arylalkoxy-, Aryloxyalkoxy-, Aryl-, Aryloxy-, Arylcarbonamidorest, einen heterocyclischen Rest oder heterocyclisch substituierten Carbonamidorest, der eine quartäre Ammoniumgruppierung enthält;

$R_4$: H, $CH_3$, $C_2H_5$;

$R_5$: H, $CH_3$;

$R_6$: H, $CH_3$;

n: 1, 2, 3, 4 oder 5

bedeutet, gegebenenfalls in Form reiner Enantiomerer oder in Form von Enantiomerengemischen, sowie die

Salze mit anorganischen und organischen Säuren.

2. Verbindungen der Formel

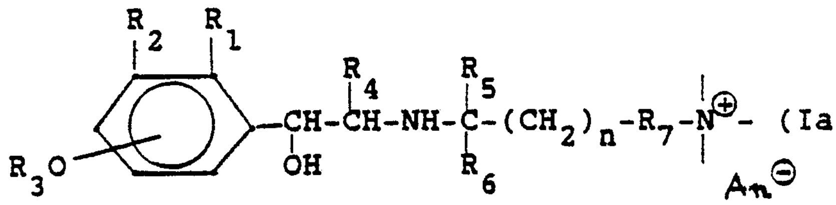 (Ia),

in der

n: 1, 2, 3, 4 oder 5;

$R_1$: H, $CH_3$, $OCH_3$, Cl, F;

$R_2$: H, $R_3O$, $CH_2OH$, NHCHO, $NHCOCH_3$, $NHSO_2CH_3$, $NHCONH_2$;

$R_3$: H, Acyl, N,N-Dialkylcarbamoyl, wobei sich die Gruppe $R_3O$ in 4- oder 5-Stellung befindet;

die Gruppe

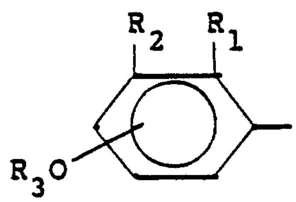 (II)

außerdem für

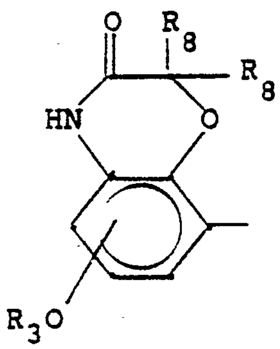 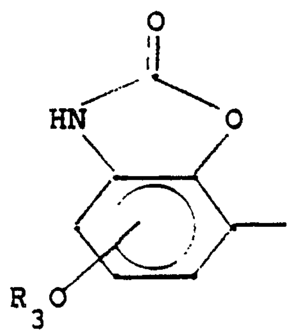 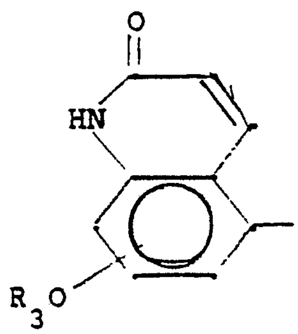

(IIa) (IIb) (IIc)

steht;

$R_4$: H, $CH_3$, $C_2H_5$;

$R_5$: H, $CH_3$;

$R_6$: H, $CH_3$;

$R_7$: eine Einfachbindung oder ein zweibindiges Brückenglied, das auch über Ringatome eines Heterocyclus an den Ammoniumstickstoff gebunden sein kann;

$R_8$: H oder $CH_3$;

$-N^+-$: eine quartäre Ammoniumgruppe.

$An^{\ominus}$: 1 Äquivalent eines Anions

bedeutet, gegebenenfalls in Form reiner Enentiomerer oder in Form von Enantiomerengemischen, sowie die Salze mit anorganischen und organischen Säuren.

3. Verbindungen nach Anspruch 2, in denen die Gruppierung $-R_7-N^{\oplus}-$ für

-E (III a)

-Ar-B-E (III b)

-O-Ar-B-E (III c)
-NH-CO-E (III d)
-NH-CO-Ar-B-E (III e)
-O-($C_mH_{2m}$)-A-E (III f)
-O-($C_mH_{2m}$)-A-Ar-B-E (III g)

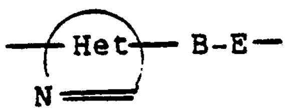
(III h)

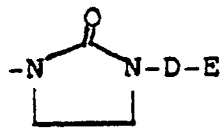
(III i)

stehen, während n und $R_1$ bis $R_6$ die obige Bedeutung haben und
m: 2, 3, 4, 5 oder 6;
A: eine Einfachbindung oder NH-CO-($C_1$-$C_4$)-Alkylen;
B: eine Einfachbindung oder -O-($C_1$-$C_4$)-Alkylen-, -NH-CO-($C_1$-$C_4$)-Alkylen-, -($C_1$-$C_4$)-Alkylen-;
D: -($C_1$-$C_4$)-Alkylen-
E: die Gruppen

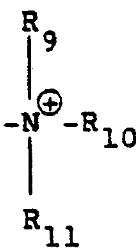
oder
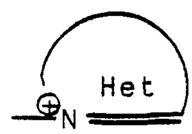
oder
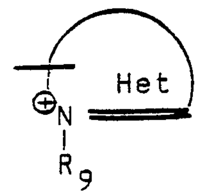

Ar: Arylen, insbesondere unsubstituiertes oder substituiertes Phenylen oder Naphthylen;

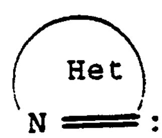

N-Heterocyclen, die mit einem Benzolring kondensiert und unsubstituiert oder substituiert sein können und die gegebenenfalls ein oder mehrere zusätzliche Heteroatome im Ring enthalten können;
$R_9$: ($C_1$-$C_4$)-Alkyl;
$R_{10}$: ($C_1$-$C_4$)-Alkyl;
$R_{11}$: ($C_1$-$C_4$)-Alkyl, ($C_1$-$C_4$)-Alkylen-$COO^{\ominus}$,
($C_1$-$C_4$)-Alkylen-$SO_3^{\ominus}$,
($C_1$-$C_4$)-Alkylen-OH, ($C_3$-$C_6$)-Cycloalkyl,
$R_9$ und $R_{10}$ gemeinsam auch ($C_4$-$C_6$)-Alkylen
bedeuten.

4. Die Verbindung der Formel

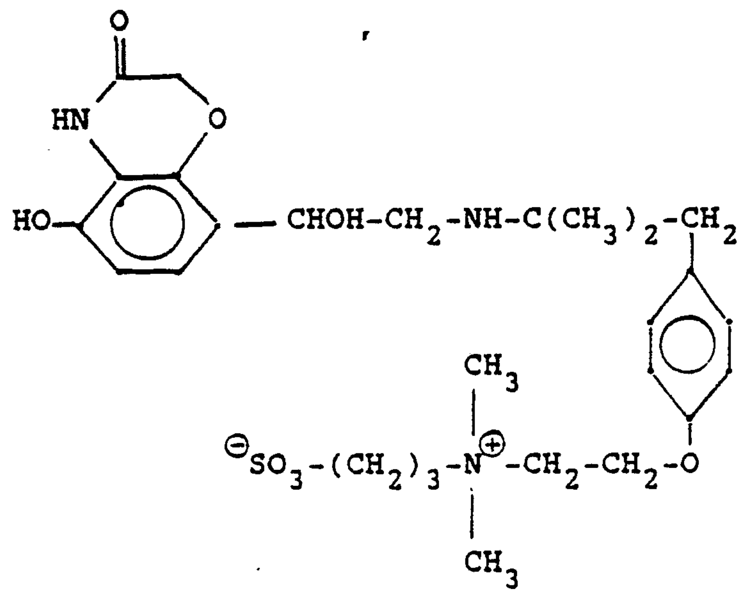

und ihre physiologisch verträglichen Säureadditionssalze.

5. Arzneimittel, gekennzeichnet durch einen Gehalt an einer Verbindung nach Anspruch 1, 2, 3 oder 4, neben an sich bekannten Hilfs- und/oder Trägerstoffen.

6. Verwendung von Verbindungen nach Anspruch 1, 2, 3 oder 4 zur Herstellung von Arzneimitteln.

7. Verwendung von therapeutisch ausreichenden Mengen von Verbindungen nach Anspruch 1, 2, 3 oder 4 bei der Behandlung von Krankheiten.

8. Verfahren zur Herstellung von Verbindungen nach Anspruch 1, 2, 3 oder 4 nach an sich bekannten Verfahren, dadurch gekennzeichnet,

a) daß man eine Verbindung der Formel

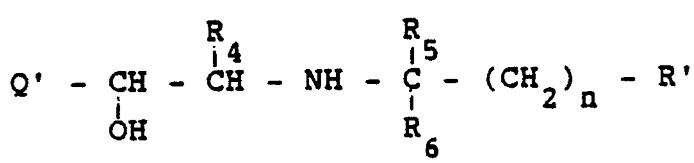 (IV),

in der n, $R_4$, $R_5$ und $R_6$ die obige Bedeutung haben und R′ für einen Rest steht, der mit R übereinstimmt, außer daß er anstelle der quartären Ammoniumgruppe eine tertiäre Aminogruppe enthält, und in der Q′ die gleiche Bedeutung wie Q hat, wobei jedoch etwa darin vorhandene OH-Gruppen, wie auch die zentrale NH-Gruppe, durch hydrogenolytisch abspaltbare Schutzgruppen geschützt sein können, mit geeigneten Alkylierungsmitteln umsetzt und gegebenenfalls vorhandene Schutzgruppen mit Wasserstoff/Katalysator entfernt oder

b) daß man zur Herstellung einer Verbindung der Formel

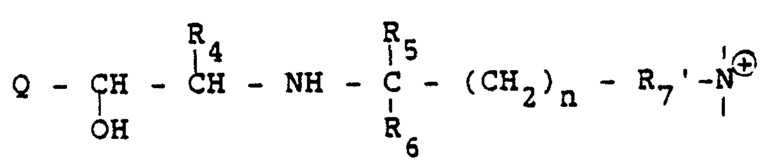 (VII),

worin die Symbole die obige Bedeutung haben, $R_7'$ eine solche Gruppe $R_7$ darstellt, die über ein aliphatisches C-Atom an den quartären Ammoniumstickstoff gebunden ist,

eine Verbindung der Formel

$$Q' - \underset{OH}{CH} - \overset{R_4}{CH} - NH - \overset{R_5}{\underset{R_6}{C}} - (CH_2)_n - R_7'-X \qquad (VIII),$$

worin die Symbole die obige Bedeutung haben und X eine Gruppe bedeutet, die unter Bindung von $R_7'$ - (und damit Quaternisierung) an das Stickstoffatom eines entsprechenden tertiären Amins abgespalten wird, mit einem tertiären Amin - $\overset{|}{N}$ -
umsetzt
oder
c) daß man aus einer Verbindung der Formel

$$Q' - CHOH - \overset{R_4}{CH} - \overset{R_{14}}{N} - \overset{R_5}{\underset{R_6}{C}} - (CH_2)_n-R \qquad (X)$$

in der $R_{14}$ für Wasserstoff oder eine hydrogenolytisch abspaltbare Schutzgruppe steht und die übrigen Symbole die oben angegebene Bedeutung haben, wobei die Verbindung X mindestens eine hydrogenolytisch abzuspaltende Gruppe enthält, mit Wasserstoff in Gegenwart eines Hydrierungskatalysators die Schutzgruppe entfernt
und daß man gewünschtenfalls die erhaltenen Verbindungen mit anorganischen oder organischen Säuren in Säureadditionssalze überführt.

9. Die Verbindungen der Formel

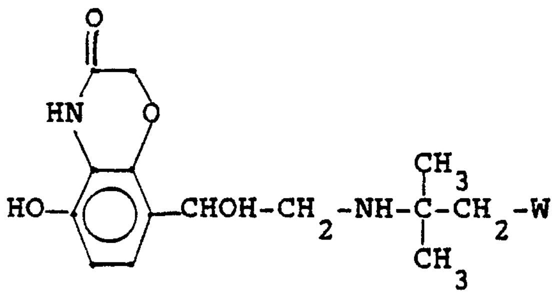 , worin

W für

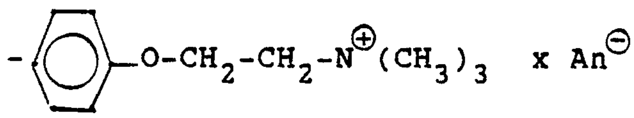

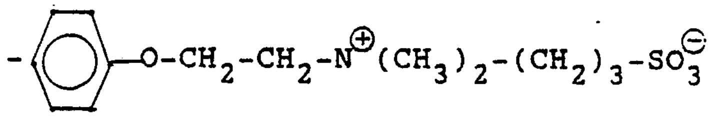

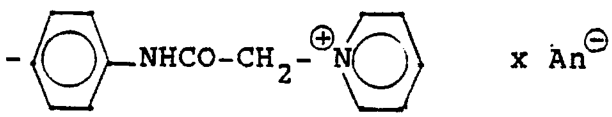

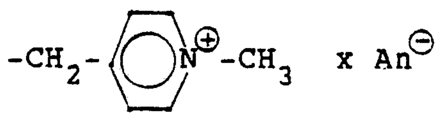

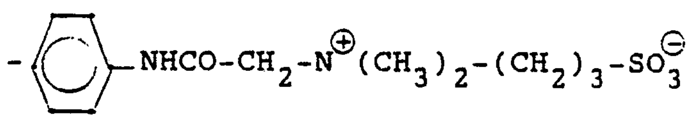

worin An$^{\ominus}$ ein Äquivalent eines Anions bedeutet, und ihre Säureadditionssalze.

10. Verwendung von Verbindungen nach Anspruch 9 bei der Herstellung von Arzneimitteln, insbesondere zur Behandlung von Bronchospasmen.